# EUROPEAN PATENT APPLICATION

(11) **EP 3 960 870 A1**
(43) Date of publication of application: **02.03.2022**
(21) Application number: 20783401.1
(22) Date of filing: 27.03.2020
(51) Int. Cl.: C12P 19/18, C12N 15/56

(54) **METHOD FOR PRODUCING ALLOLACTOSE**

(30) Priority: 29.03.2019 JP 2019068796
(71) Applicant: AJINOMOTO CO., INC., Chuo-ku Tokyo 104-8315 (JP)
(72) Inventor: YAHAGI, Daiki, Kawasaki-shi, Kanagawa 210-8681 (JP)
(74) Representative: Strehl Schübel-Hopf & Partner
(86) International application number: PCT/JP2020/014313
(87) International publication number: WO 2020/203885

(57) **Abstract**

A method for producing allolactose is provided. Allolactose is produced by bringing cells of a microorganism having beta-galactosidase (BGL) into contact with lactose in the presence of a substance that inhibits hydrolysis of allolactose.

SEQUENCE LISTING

<110>  Ajinomoto Co., Inc.

<120>  METHOD FOR PRODUCING ALLOLACTOSE

<130>  F250-19654

<150>  JP2019-068796
<151>  2019-03-29

<160>  16

<170>  PatentIn version 3.5

<210>  1
<211>  43
<212>  DNA
<213>  Artificial Sequence

<220>
<223>  primer

<400>  1
aagaaggaga tatacatgac catgattacg gattcactgg ccg                43

<210>  2
<211>  71
<212>  DNA
<213>  Artificial Sequence

<220>
<223>  primer

<400>  2
gttagcagcc ggatcttaat ggtgatggtg atggtgtttt tgacaccaga ccaactggta      60
atggtagcga c                                                           71

<210>  3
<211>  43
<212>  DNA
<213>  Artificial Sequence

<220>
<223>  primer

<400>  3
gtatatctcc ttcttaaagt taaacaaaat tatttctaga ggg                43

<210>  4
<211>  23
<212>  DNA
<213>  Artificial Sequence

<220>
<223>  primer

<400>  4
gatccggctg ctaacaaagc ccg                                      23

<210>  5
<211>  43
<212>  DNA
<213>  Artificial Sequence

<220>
<223>  primer

<400>  5
acacaggaaa cagctatgac ggcattgacg gaaggtgcaa aac                    43


<210>  6
<211>  42
<212>  DNA
<213>  Artificial Sequence

<220>
<223>  primer

<400>  6
gggctggctt aactattact gatacgtgtc cagatcaacc gc                     42


<210>  7
<211>  32
<212>  DNA
<213>  Artificial Sequence

<220>
<223>  primer

<400>  7
agctgtttcc tgtgtgaaat tgttatccgc tc                                32


<210>  8
<211>  22
<212>  DNA
<213>  Artificial Sequence

<220>
<223>  primer

<400>  8
tagttaagcc agccccgaca cc                                           22


<210>  9
<211>  69
<212>  DNA
<213>  Artificial Sequence

<220>
<223>  primer

<400>  9
aatgtgtgga attgtgagcg gataacaatt tcacacagga atgaaaccag taacgttata    60
cgatgtcgc                                                          69


<210>  10

<211>  36
<212>  DNA
<213>  Artificial Sequence

<220>
<223>  primer

<400>  10
ggtggcactt ttcggtcact gcccgctttc cagtcg                                36


<210>  11
<211>  20
<212>  DNA
<213>  Artificial Sequence

<220>
<223>  primer

<400>  11
ccgaaaagtg ccacctgacg                                                  20


<210>  12
<211>  67
<212>  DNA
<213>  Artificial Sequence

<220>
<223>  primer

<400>  12
acaattccac acattatacg agccgatgat taattgtaca caggggggga aatgtgcgcg      60
gaacccc                                                                67


<210>  13
<211>  42
<212>  DNA
<213>  Artificial Sequence

<220>
<223>  primer

<400>  13
aagaaggaga tatacatggg tagcgtaact agtaccaacg gc                         42


<210>  14
<211>  73
<212>  DNA
<213>  Artificial Sequence

<220>
<223>  primer

<400>  14
gttagcagcc ggatctcagt ggtggtggtg gtggtgctct ttcgagatgt atttgttgaa      60
caattctcca atg                                                         73


<210>  15
<211>  3075

<212>  DNA
<213>  Escherichia coli

<400>  15
atgaccatga ttacggattc actggccgtc gttttacaac gtcgtgactg ggaaaaccct      60
ggcgttaccc aacttaatcg ccttgcagca catccccctt tcgccagctg gcgtaatagc     120
gaagaggccc gcaccgatcg cccttcccaa cagttgcgca gcctgaatgg cgaatggcgc     180
tttgcctggt ttccggcacc agaagcggtg ccggaaagct ggctggagtg cgatcttcct     240
gaggccgata ctgtcgtcgt cccctcaaac tggcagatgc acggttacga tgcgcccatc     300
tacaccaacg tgacctatcc cattacggtc aatccgccgt ttgttcccac ggagaatccg     360
acgggttgtt actcgctcac atttaatgtt gatgaaagct ggctacagga aggccagacg     420
cgaattattt ttgatggcgt taactcggcg tttcatctgt ggtgcaacgg gcgctgggtc     480
ggttacggcc aggacagtcg tttgccgtct gaatttgacc tgagcgcatt tttacgcgcc     540
ggagaaaacc gcctcgcggt gatggtgctg cgctggagtg acggcagtta tctggaagat     600
caggatatgt ggcggatgag cggcattttc cgtgacgtct cgttgctgca taaaccgact     660
acacaaatca gcgatttcca tgttgccact cgctttaatg atgatttcag ccgcgctgta     720
ctggaggctg aagttcagat gtgcggcgag ttgcgtgact acctacgggt aacagtttct     780
ttatggcagg gtgaaacgca ggtcgccagc ggcaccgcgc ctttcggcgg tgaaattatc     840
gatgagcgtg gtggttatgc cgatcgcgtc acactacgtc tgaacgtcga aaacccgaaa     900
ctgtggagcg ccgaaatccc gaatctctat cgtgcggtgg ttgaactgca caccgccgac     960
ggcacgctga ttgaagcaga agcctgcgat gtcggtttcc gcgaggtgcg gattgaaaat    1020
ggtctgctgc tgctgaacgg caagccgttg ctgattcgag gcgttaaccg tcacgagcat    1080
catcctctgc atggtcaggt catggatgag cagacgatgg tgcaggatat cctgctgatg    1140
aagcagaaca actttaacgc cgtgcgctgt tcgcattatc cgaaccatcc gctgtggtac    1200
acgctgtgcg accgctacgg cctgtatgtg gtggatgaag ccaatattga aacccacggc    1260
atggtgccaa tgaatcgtct gaccgatgat ccgcgctggc taccggcgat gagcgaacgc    1320
gtaacgcgaa tggtgcagcg cgatcgtaat cacccgagtg tgatcatctg gtcgctgggg    1380
aatgaatcag gccacggcgc taatcacgac gcgctgtatc gctggatcaa atctgtcgat    1440
ccttcccgcc cggtgcagta tgaaggcggc ggagccgaca ccacggccac cgatattatt    1500
tgcccgatgt acgcgcgcgt ggatgaagac cagcccttcc cggctgtgcc gaaatggtcc    1560
atcaaaaaat ggctttcgct acctggagag acgcgcccgc tgatcctttg cgaatacgcc    1620
cacgcgatgg gtaacagtct tggcggtttc gctaaatact ggcaggcgtt tcgtcagtat    1680
ccccgtttac agggcggctt cgtctgggac tgggtggatc agtcgctgat taaatatgat    1740
gaaaacggca acccgtggtc ggcttacggc ggtgattttg gcgatacgcc gaacgatcgc    1800
cagttctgta tgaacggtct ggtctttgcc gaccgcacgc cgcatccagc gctgacggaa    1860
gcaaaacacc agcagcagtt tttccagttc cgtttatccg ggcaaaccat cgaagtgacc    1920
agcgaatacc tgttccgtca tagcgataac gagctcctgc actggatggt ggcgctggat    1980
ggtaagccgc tggcaagcgg tgaagtgcct ctggatgtcg ctccacaagg taaacagttg    2040
attgaactgc ctgaactacc gcagccggag agcgccgggc aactctggct cacagtacgc    2100
gtagtgcaac cgaacgcgac cgcatggtca gaagccgggc acatcagcgc ctggcagcag    2160
tggcgtctgg cggaaaacct cagtgtgacg ctccccgccg cgtcccacgc catcccgcat    2220
ctgaccacca gcgaaatgga tttttgcatc gagctgggta ataagcgttg gcaatttaac    2280
cgccagtcag gctttctttc acagatgtgg attggcgata aaaaacaact gctgacgccg    2340
ctgcgcgatc agttcacccg tgcaccgctg gataacgaca ttggcgtaag tgaagcgacc    2400
cgcattgacc ctaacgcctg ggtcgaacgc tggaaggcgg cgggccatta ccaggccgaa    2460
gcagcgttgt tgcagtgcac ggcagataca cttgctgatg cggtgctgat tacgaccgct    2520
cacgcgtggc agcatcaggg gaaaacctta tttatcagcc ggaaaaccta ccggattgat    2580
ggtagtggtc aaatggcgat taccgttgat gttgaagtgg cgagcgatac accgcatccg    2640
gcgcggattg gcctgaactg ccagctggcg caggtagcag agcgggtaaa ctggctcgga    2700
ttagggccgc aagaaaacta tcccgaccgc cttactgccg cctgttttga ccgctgggat    2760
ctgccattgt cagacatgta taccccgtac gtcttcccga gcgaaaacgg tctgcgctgc    2820
gggacgcgcg aattgaatta tggcccacac cagtggcgcg gcgacttcca gttcaacatc    2880
agccgctaca gtcaacagca actgatggaa accagccatc gccatctgct gcacgcggaa    2940
gaaggcacat ggctgaatat cgacggtttc catatgggga ttggtggcga cgactcctgg    3000
agcccgtcag tatcggcgga attccagctg agcgccggtc gctaccatta ccagttggtc    3060
tggtgtcaaa aataa                                                     3075

<210>  16
<211>  1024
<212>  PRT
<213>  Escherichia coli

<400> 16
Met Thr Met Ile Thr Asp Ser Leu Ala Val Val Leu Gln Arg Arg Asp
1               5                   10                  15
Trp Glu Asn Pro Gly Val Thr Gln Leu Asn Arg Leu Ala Ala His Pro
            20                  25                  30
Pro Phe Ala Ser Trp Arg Asn Ser Glu Glu Ala Arg Thr Asp Arg Pro
        35                  40                  45
Ser Gln Gln Leu Arg Ser Leu Asn Gly Glu Trp Arg Phe Ala Trp Phe
    50                  55                  60
Pro Ala Pro Glu Ala Val Pro Glu Ser Trp Leu Glu Cys Asp Leu Pro
65                  70                  75                  80
Glu Ala Asp Thr Val Val Val Pro Ser Asn Trp Gln Met His Gly Tyr
                85                  90                  95
Asp Ala Pro Ile Tyr Thr Asn Val Thr Tyr Pro Ile Thr Val Asn Pro
            100                 105                 110
Pro Phe Val Pro Thr Glu Asn Pro Thr Gly Cys Tyr Ser Leu Thr Phe
        115                 120                 125
Asn Val Asp Glu Ser Trp Leu Gln Glu Gly Gln Thr Arg Ile Ile Phe
    130                 135                 140
Asp Gly Val Asn Ser Ala Phe His Leu Trp Cys Asn Gly Arg Trp Val
145                 150                 155                 160
Gly Tyr Gly Gln Asp Ser Arg Leu Pro Ser Glu Phe Asp Leu Ser Ala
                165                 170                 175
Phe Leu Arg Ala Gly Glu Asn Arg Leu Ala Val Met Val Leu Arg Trp
            180                 185                 190
Ser Asp Gly Ser Tyr Leu Glu Asp Gln Asp Met Trp Arg Met Ser Gly
        195                 200                 205
Ile Phe Arg Asp Val Ser Leu Leu His Lys Pro Thr Thr Gln Ile Ser
    210                 215                 220
Asp Phe His Val Ala Thr Arg Phe Asn Asp Asp Phe Ser Arg Ala Val
225                 230                 235                 240
Leu Glu Ala Glu Val Gln Met Cys Gly Glu Leu Arg Asp Tyr Leu Arg
                245                 250                 255
Val Thr Val Ser Leu Trp Gln Gly Glu Thr Gln Val Ala Ser Gly Thr
            260                 265                 270
Ala Pro Phe Gly Gly Glu Ile Ile Asp Glu Arg Gly Gly Tyr Ala Asp
        275                 280                 285
Arg Val Thr Leu Arg Leu Asn Val Glu Asn Pro Lys Leu Trp Ser Ala
    290                 295                 300
Glu Ile Pro Asn Leu Tyr Arg Ala Val Val Glu Leu His Thr Ala Asp
305                 310                 315                 320
Gly Thr Leu Ile Glu Ala Glu Ala Cys Asp Val Gly Phe Arg Glu Val
                325                 330                 335
Arg Ile Glu Asn Gly Leu Leu Leu Leu Asn Gly Lys Pro Leu Leu Ile
            340                 345                 350
Arg Gly Val Asn Arg His Glu His His Pro Leu His Gly Gln Val Met
        355                 360                 365
Asp Glu Gln Thr Met Val Gln Asp Ile Leu Leu Met Lys Gln Asn Asn
    370                 375                 380
Phe Asn Ala Val Arg Cys Ser His Tyr Pro Asn His Pro Leu Trp Tyr
385                 390                 395                 400
Thr Leu Cys Asp Arg Tyr Gly Leu Tyr Val Val Asp Glu Ala Asn Ile
                405                 410                 415
Glu Thr His Gly Met Val Pro Met Asn Arg Leu Thr Asp Asp Pro Arg
            420                 425                 430
Trp Leu Pro Ala Met Ser Glu Arg Val Thr Arg Met Val Gln Arg Asp
        435                 440                 445
Arg Asn His Pro Ser Val Ile Ile Trp Ser Leu Gly Asn Glu Ser Gly
    450                 455                 460
His Gly Ala Asn His Asp Ala Leu Tyr Arg Trp Ile Lys Ser Val Asp
465                 470                 475                 480
Pro Ser Arg Pro Val Gln Tyr Glu Gly Gly Gly Ala Asp Thr Thr Ala
                485                 490                 495

Thr Asp Ile Ile Cys Pro Met Tyr Ala Arg Val Asp Glu Asp Gln Pro
                500                 505                 510
Phe Pro Ala Val Pro Lys Trp Ser Ile Lys Lys Trp Leu Ser Leu Pro
        515                 520                 525
Gly Glu Thr Arg Pro Leu Ile Leu Cys Glu Tyr Ala His Ala Met Gly
    530                 535                 540
Asn Ser Leu Gly Gly Phe Ala Lys Tyr Trp Gln Ala Phe Arg Gln Tyr
545                 550                 555                 560
Pro Arg Leu Gln Gly Gly Phe Val Trp Asp Trp Val Asp Gln Ser Leu
                565                 570                 575
Ile Lys Tyr Asp Glu Asn Gly Asn Pro Trp Ser Ala Tyr Gly Gly Asp
            580                 585                 590
Phe Gly Asp Thr Pro Asn Asp Arg Gln Phe Cys Met Asn Gly Leu Val
        595                 600                 605
Phe Ala Asp Arg Thr Pro His Pro Ala Leu Thr Glu Ala Lys His Gln
    610                 615                 620
Gln Gln Phe Phe Gln Phe Arg Leu Ser Gly Gln Thr Ile Glu Val Thr
625                 630                 635                 640
Ser Glu Tyr Leu Phe Arg His Ser Asp Asn Glu Leu Leu His Trp Met
                645                 650                 655
Val Ala Leu Asp Gly Lys Pro Leu Ala Ser Gly Glu Val Pro Leu Asp
            660                 665                 670
Val Ala Pro Gln Gly Lys Gln Leu Ile Glu Leu Pro Glu Leu Pro Gln
        675                 680                 685
Pro Glu Ser Ala Gly Gln Leu Trp Leu Thr Val Arg Val Val Gln Pro
    690                 695                 700
Asn Ala Thr Ala Trp Ser Glu Ala Gly His Ile Ser Ala Trp Gln Gln
705                 710                 715                 720
Trp Arg Leu Ala Glu Asn Leu Ser Val Thr Leu Pro Ala Ala Ser His
                725                 730                 735
Ala Ile Pro His Leu Thr Thr Ser Glu Met Asp Phe Cys Ile Glu Leu
            740                 745                 750
Gly Asn Lys Arg Trp Gln Phe Asn Arg Gln Ser Gly Phe Leu Ser Gln
        755                 760                 765
Met Trp Ile Gly Asp Lys Lys Gln Leu Leu Thr Pro Leu Arg Asp Gln
    770                 775                 780
Phe Thr Arg Ala Pro Leu Asp Asn Asp Ile Gly Val Ser Glu Ala Thr
785                 790                 795                 800
Arg Ile Asp Pro Asn Ala Trp Val Glu Arg Trp Lys Ala Ala Gly His
                805                 810                 815
Tyr Gln Ala Glu Ala Ala Leu Leu Gln Cys Thr Ala Asp Thr Leu Ala
            820                 825                 830
Asp Ala Val Leu Ile Thr Thr Ala His Ala Trp Gln His Gln Gly Lys
        835                 840                 845
Thr Leu Phe Ile Ser Arg Lys Thr Tyr Arg Ile Asp Gly Ser Gly Gln
    850                 855                 860
Met Ala Ile Thr Val Asp Val Glu Val Ala Ser Asp Thr Pro His Pro
865                 870                 875                 880
Ala Arg Ile Gly Leu Asn Cys Gln Leu Ala Gln Val Ala Glu Arg Val
                885                 890                 895
Asn Trp Leu Gly Leu Gly Pro Gln Glu Asn Tyr Pro Asp Arg Leu Thr
            900                 905                 910
Ala Ala Cys Phe Asp Arg Trp Asp Leu Pro Leu Ser Asp Met Tyr Thr
        915                 920                 925
Pro Tyr Val Phe Pro Ser Glu Asn Gly Leu Arg Cys Gly Thr Arg Glu
    930                 935                 940
Leu Asn Tyr Gly Pro His Gln Trp Arg Gly Asp Phe Gln Phe Asn Ile
945                 950                 955                 960
Ser Arg Tyr Ser Gln Gln Gln Leu Met Glu Thr Ser His Arg His Leu
                965                 970                 975
Leu His Ala Glu Glu Gly Thr Trp Leu Asn Ile Asp Gly Phe His Met
            980                 985                 990
Gly Ile Gly Gly Asp Asp Ser Trp  Ser Pro Ser Val Ser  Ala Glu Phe

995                 1000                1005
Gln Leu Ser Ala Gly Arg Tyr His Tyr Gln Leu Val Trp Cys Gln
    1010                1015                1020
Lys

## Description

### Technical Field

The present invention relates to a method for producing allolactose.

Expression of lac operon (i.e. gene expression from lac promoter) is induced in the presence of lactose. The true expression inducer of lac promoter is allolactose, which is an isomer of lactose. Allolactose is produced, for example, from lactose by catalytic action of beta-galactosidase (BGL). Examples of BGL include lacZ protein encoded by *lacZ* gene of lac operon.

It has been reported that a galactooligosaccharide mixture containing allolactose was prepared from a high concentration of lactose using a microbial BGL as a purified enzyme or enzymatic cells (Non-patent documents 1-4).

In the field of biotechnology, isopropyl-beta-thiogalactopyranoside (IPTG), which is an analog of lactose, is used as a versatile inducer of lac promoter.

### Prior art references

### Non-patent documents

Non-patent document 1: Claudia VY et al., Galactooligosaccharide Production from Pantoea anthophila Strains Isolated from "Tejuino", a Mexican Traditional Fermented Beverage. Catalysts 2017, 7(8), 242.
Non-patent document 2: Arreola SL et al., Two β-galactosidases from the human isolate Bifidobacterium breve DSM 20213: molecular cloning and expression, biochemical characterization and synthesis of galacto-oligosaccharides. PLoS One. 2014 Aug 4;9(8):e104056.
Non-patent document 3: Urrutia P et al., Detailed analysis of galactooligosaccharides synthesis with β-galactosidase from Aspergillus oryzae. J Agric Food Chem. 2013 Feb 6;61(5): 1081-7.
Non-patent document 4: Rodriguez-Colinas B et al., Production of Galacto-oligosaccharides by the β-Galactosidase from Kluyveromyces lactis: comparative analysis of permeabilized cells versus soluble enzyme. J Agric Food Chem. 2011 Oct 12;59(19): 10477-84.

### Summary of the Invention

### Object to be Achieved by the Invention

An object of the present invention is to provide a method for producing allolactose.

### Means for Achieving the Object

In order to achieve the aforementioned object, the inventors of the present invention conducted various researches. As a result, they found that allolactose can be efficiently produced by allowing *Escherichia coli* cells having beta-galactosidase (BGL) to act on a high concentration of lactose in the presence of a high concentration of glucose, and accomplished the present invention.

The present invention can be thus embodied, for example, as follows.
[1] A method for producing allolactose, the method comprising a step (A) mentioned below:
   (A) a step of bringing cells of a microorganism having beta-galactosidase into contact with lactose in the presence of a substance X that inhibits hydrolysis of allolactose,
      wherein the cells have not been subject to a treatment for increasing membrane permeability, and
      wherein the total concentration of lactose and the substance X is 1 Eq/L or more.
[2] A method for producing a gene expression inducer, the method comprising a step (A) mentioned below:
   (A) a step of bringing cells of a microorganism having beta-galactosidase into contact with lactose in the presence of a substance X that inhibits hydrolysis of allolactose,
      wherein the cells have not been subject to a treatment for increasing membrane permeability, and
      wherein the total concentration of lactose and the substance X is 1 Eq/L or more.
[3] The method mentioned above, wherein the substance X is glucose and/or galactose.
[4] The method mentioned above, wherein the substance X is glucose.
[5] The method mentioned above, wherein the concentration of lactose in the step (A) is 120 g/L or more.
[6] The method mentioned above, wherein the concentration of the substance X in the step (A) is 120 g/L or more.
[7] The method mentioned above, wherein the step (A) is carried out at a temperature of 30°C or below.
[8] The method mentioned above, wherein the cells have not been subject to any of drying treatment, freeze-thaw treatment, surfactant treatment, and organic solvent treatment.
[9] The method mentioned above, wherein the microorganism is *Escherichia coli.*
[10] The method mentioned above, wherein the microorganism has been modified so that the activity of beta-galactosidase is increased as compared with a non-modified strain.
[11] The method mentioned above, wherein the beta-galactosidase is a protein defined in (a), (b), or (c) mentioned below:
   (a) a protein comprising the amino acid sequence of SEQ ID NO: 16;
   (b) a protein comprising the amino acid sequence of SEQ ID NO: 16, but which includes substitution, deletion, insertion, and/or addition of 1 to 10 amino acid residues, and having allolactose-generating activity and allolactose-hydrolyzing activity;
   (c) a protein comprising an amino acid sequence showing an identity of 90% or higher to the amino acid sequence of SEQ ID NO: 16, and having allolactose-generating activity and allolactose-hydrolyzing activity.

### Brief Description of Drawings

[FIG. 1] A diagram showing a result of allolactose production from 2% lactose using LacZ purified enzyme.
[FIG. 2] A diagram showing a result of allolactose production from 20% lactose using LacZ purified enzyme.
[FIG. 3] A diagram showing a result of allolactose production from 20% lactose in the presence of 10% glucose using LacZ purified enzyme.
[FIG. 4] A diagram showing a result of allolactose production from 20% lactose in the presence of 20% glucose using LacZ purified enzyme.
[FIG. 5] A diagram showing a result of allolactose production from various saccharide solutions using *Escherichia coli* viable cells having LacZ.
[FIG. 6] A diagram showing an effect of temperature on allolactose production from 20% lactose in the presence of 20% glucose using *Escherichia coli* viable cells having LacZ.
[FIG. 7] A diagram (photograph) showing a result of induced expression of DasherGFP from lac promoter using prepared allolactose.
[FIG. 8] A diagram (photograph) showing a result of induced expression of GH1-2 protein via induced expression of T7-polymerase from lac promoter using prepared allolactose.

### Modes for Carrying out the Invention

Hereinafter, the present invention will be explained in detail.

The method of the present invention is a method for producing allolactose, the method comprising a step of bringing cells of a microorganism having beta-galactosidase (BGL) into contact with lactose in the presence of a substance that inhibits hydrolysis of allolactose. This step is also referred to as "conversion step" or "conversion reaction". This microorganism is also referred to as "microorganism of the present invention". These cells are also referred to as "cells of the present invention". The substance that inhibits hydrolysis of allolactose is also referred to as "substance X".

The conversion step results in generation of allolactose. That is, the conversion step may be a step of generating allolactose by bringing cells of the present invention into contact with lactose in the presence of the substance X. The conversion step specifically results in generation of allolactose from lactose. That is, the conversion step may specifically be a step of generating allolactose from lactose by bringing cells of the present invention into contact with lactose in the presence of the substance X.

### <1> The microorganism of the present invention and the cells of the present invention <1-1> The microorganism of the present invention

The microorganism of the present invention is a microorganism having beta-galactosidase (BGL). The phrase "a microorganism has BGL" means specifically that a microorganism has BGL in cells of the microorganism. In other words, the cells of the present invention have BGL. The microorganism of the present invention (i.e. the cells of the present invention) may have one kind of BGL or two or more kinds of BGLs.

BGL is expressed from a gene encoding BGL. The gene encoding BGL is also referred to as "beta-galactosidase gene (BGL gene)". Hence, the microorganism of the present invention has a BGL gene. The microorganism of the present invention specifically has a BGL gene so that the BGL gene can be expressed. The microorganism of the present invention may or may not be a microorganism inherently having a BGL gene. The microorganism of the present invention may be, for example, a microorganism inherently not having a BGL gene but modified so as to have a BGL gene. A microorganism modified so as to have a BGL gene can be obtained by introducing the BGL gene into a microorganism not having the BGL gene. Means for introducing a gene will be described below. The microorganism of the present invention may have one kind of BGL gene or two or more kinds of BGL genes. The microorganism of the present invention may have one copy of a BGL gene or two or more copies of a BGL gene.

The microorganism of the present invention may have been modified so that the activity of BGL is increased. The microorganism of the present invention may have been modified specifically so that the activity of BGL is increased as compared with a non-modified strain. For example, a microorganism inherently not having a BGL gene may be modified so that the activity of BGL is increased. That is, by introducing a BGL gene into a microorganism not having a BGL gene, BGL activity of the microorganism can be increased, i.e. the microorganism can be imparted with BGL activity. Alternatively, for example, a microorganism inherently having a BGL gene may be modified so that the activity of BGL is increased. The phrase "the activity of BGL is increased" may mean that at least the allolactose-generating activity is increased. The phrase "the activity of BGL is increased" may typically mean that both the allolactose-generating activity and the allolactose-hydrolyzing activity are increased. Means for increasing the activity of a protein such as an enzyme will be described below.

As the microorganism of the present invention, such a microorganism as described below may be used as it is, or after subject to modification, such as introduction of a BGL gene and enhancement of BGL activity, as required. That is, the microorganism of the present invention may be a modified strain derived from such a microorganism as described below. The microorganism of the present invention or a parent strain for constructing the same is also referred to as "host".

The microorganism used as the microorganism of the present invention or a parent strain for constructing the same is not particularly limited. Examples of the microorganism include bacteria and yeast.

Examples of bacteria include bacteria belonging to the family *Enterobacteriaceae* and coryneform bacteria.

Examples of bacteria belonging to the family *Enterobacteriaceae* include bacteria belonging to the genus *Escherichia, Enterobacter, Pantoea, Klebsiella, Serratia, Erwinia, Photorhabdus, Providencia, Salmonella, Morganella,* or the like. Specifically, bacteria classified into the family *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used.

The *Escherichia* bacteria are not particularly limited, and examples thereof include those classified into the genus *Escherichia* according to the taxonomy known to those skilled in the field of microbiology. Examples of the *Escherichia* bacteria include, for example, those described in the work of Neidhardt et al. (Backmann B.J., 1996, Derivations and Genotypes of some mutant derivatives of Escherichia coli K-12, pp.2460-2488, Table 1, In F.D. Neidhardt (ed.), Escherichia coli and Salmonella Cellular and Molecular Biology/Second Edition, American Society for Microbiology Press, Washington, D.C.). Examples of the *Escherichia* bacteria include, for example, *Escherichia coli.* Specific examples of *Escherichia coli* include, for example, *Escherichia coli* K-12 strains such as W3110 strain (ATCC 27325) and MG1655 strain (ATCC 47076); *Escherichia coli* K5 strain (ATCC 23506); *Escherichia coli* B strains such as BL21 (DE3) strain; and derivative strains thereof.

The *Enterobacter* bacteria are not particularly limited, and examples include those classified into the genus *Enterobacter* according to the taxonomy known to those skilled in the field of microbiology. Examples of the *Enterobacter* bacterium include, for example, *Enterobacter agglomerans* and *Enterobacter aerogenes.* Specific examples of *Enterobacter agglomerans* include, for example, the *Enterobacter agglomerans* ATCC 12287 strain. Specific examples of *Enterobacter aerogenes* include, for example, the *Enterobacter aerogenes* ATCC 13048 strain, NBRC 12010 strain (Biotechnol. Bioeng., 2007, Mar. 27;98(2):340-348), and AJ110637 strain (FERM BP-10955). Examples of the *Enterobacter* bacteria also include, for example, the strains described in European Patent Application Laid-open (EP-A) No. 0952221. In addition, *Enterobacter agglomerans* also include some strains classified as *Pantoea agglomerans.*

The *Pantoea* bacteria are not particularly limited, and examples include those classified into the genus *Pantoea* according to the taxonomy known to those skilled in the field of microbiology. Examples of the *Pantoea* bacteria include, for example, *Pantoea ananatis, Pantoea stewartii, Pantoea agglomerans,* and *Pantoea citrea.* Specific examples of *Pantoea ananatis* include, for example, the *Pantoea ananatis* LMG20103 strain, AJ13355 strain (FERM BP-6614), AJ13356 strain (FERM BP-6615), AJ13601 strain (FERM BP-7207), SC17 strain (FERM BP-11091), SC17(0) strain (VKPM B-9246), and SC17sucA strain (FERM BP-8646). Some of *Enterobacter* bacteria and *Erwinia* bacteria were reclassified into the genus *Pantoea* (Int. J. Syst. Bacteriol., 39, 337-345 (1989); Int. J. Syst. Bacteriol., 43, 162-173 (1993)). For example, some strains of *Enterobacter agglomerans* were recently reclassified into *Pantoea agglomerans, Pantoea ananatis, Pantoea stewartii,* or the like on the basis of nucleotide sequence analysis of 16S rRNA etc. (Int. J. Syst. Bacteriol., 39, 337-345 (1989)). The *Pantoea* bacteria may include those reclassified into the genus *Pantoea* as described above.

Examples of the *Erwinia* bacteria include *Erwinia amylovora* and *Erwinia carotovora.* Examples of the *Klebsiella* bacteria include *Klebsiella planticola.*

Examples of the coryneform bacteria include bacteria belonging to the genus *Corynebacterium, Brevibacterium, Microbacterium,* or the like.

Specific examples of the coryneform bacteria include the following species.
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*

Specific examples of the coryneform bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens (Corynebacterium thermoaminogenes*) AJ12340 (FERM BP-1539)
*Corynebacterium herculis* ATCC 13868
*Brevibacterium divaricatum (Corynebacterium glutamicum)* ATCC 14020
*Brevibacterium flavum (Corynebacterium glutamicum)* ATCC 13826, ATCC 14067, AJ12418 (FERM BP-2205)
*Brevibacterium immariophilum* ATCC 14068
*Brevibacterium lactofermentum (Corynebacterium glutamicum)* ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes (Corynebacterium stationis)* ATCC 6871, ATCC 6872
*Brevibacterium album* ATCC 15111
*Brevibacterium cerinum* ATCC 15112
*Microbacterium ammoniaphilum* ATCC 15354

The *Corynebacterium* bacteria include bacteria that had previously been classified into the genus *Brevibacterium,* but are presently united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but are presently re-classified into *Corynebacterium stationis* on the basis of nucleotide sequence analysis of 16S rRNA etc. (Int. J. Syst. Evol. Microbiol., 60, 874-879 (2010)).

The yeast may be budding yeast or may be fission yeast. The yeast may be haploid yeast or may be diploid or more polyploid yeast. Examples of the yeast include yeast belonging to the genus *Saccharomyces* such as *Saccharomyces cerevisiae,* the genus *Pichia* (also referred to as the genus *Wickerhamomyces)* such as *Pichia ciferrii, Pichia sydowiorum,* and *Pichia pastoris,* the genus *Candida* such as *Candida utilis,* the genus *Hansenula* such as *Hansenula polymorpha,* and the genus *Schizosaccharomyces* such as *Schizosaccharomyces pombe.*

These strains are available from, for example, the American Type Culture Collection (Address: 12301 Parklawn Drive, Rockville, Maryland 20852, P.O. Box 1549, Manassas, VA20108, United States of America). That is, registration numbers are given to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

### <1-2> Beta-galactosidase (BGL)

The phrase "beta-galactosidase (BGL)" refers to a protein having an activity of catalyzing the reaction of isomerizing lactose to thereby produce allolactose and an activity of catalyzing the reaction of hydrolyzing allolactose to thereby produce glucose and galactose. The former activity is also referred to as "allolactose-generating activity". The latter activity is also referred to as "allolactose-hydrolyzing activity". BGL may also have an activity of catalyzing the reaction of hydrolyzing lactose to thereby produce glucose and galactose. This activity is also referred to as "lactose-hydrolyzing activity". A gene encoding BGL is also referred to as "beta-galactosidase gene (BGL gene)". Examples of BGL include LacZ protein encoded by *lacZ* gene. Specific examples of BGL such as LacZ protein include those of *Enterobacteriaceae* bacteria (e.g. *Escherichia* bacteria such as *E. coli*) and coryneform bacteria (e.g. *Corynebacterium* bacteria such as C. *glutamicum*)*.* The nucleotide sequence of the *lacZ* gene *of E. coli* K-12 MG1655 and the amino acid sequence of the LacZ protein encoded by the gene are shown in SEQ ID NOS: 15 and 16, respectively.

That is, the BGL gene may be, for example, a gene having a known nucleotide sequence such as the nucleotide sequence exemplified above. Also, BGL gene may be, for example, a protein having a known amino acid sequence such as the amino acid sequence exemplified above. The phrase "having a (nucleotide or amino acid) sequence" means comprising the (nucleotide or amino acid) sequence unless otherwise stated, and also includes cases of consisting of the (nucleotide or amino acid) sequence.

The BGL gene may be a variant of the BGL gene exemplified above (e.g. a variant of a gene having a known nucleotide sequence such as the nucleotide sequence exemplified above), so long as the original function thereof is maintained. Similarly, BGL may be a variant of the BGL exemplified above (e.g. a variant of a protein having a known amino acid sequence such as the amino acid sequence exemplified above), so long as the original function thereof is maintained. Such a variant that maintains the original function thereof is also referred to as "conservative variant". A gene defined with the aforementioned gene name and a protein defined with the aforementioned protein name can include not only the genes and proteins exemplified above, respectively, but may also include conservative variants thereof. Namely, for example, the phrase "*lacZ* gene" includes not only the *lacZ* gene exemplified above (e.g. a gene having the nucleotide sequence shown as SEQ ID NO: 15), but may also include conservative variants thereof. Similarly, for example, the phrase "LacZ protein" includes not only the LacZ protein exemplified above (e.g. a protein having the amino acid sequence shown as SEQ ID NO: 16), but may also include conservative variants thereof. Examples of the conservative variants include, for example, homologues and artificially modified versions of the genes and proteins exemplified above.

The phrase "the original function is maintained" means that a variant of a gene or protein has a function (such as activity or property) corresponding to the function (such as activity or property) of the original gene or protein. That is, the phrase "the original function is maintained" used for the BGL gene means that a variant of the gene encodes BGL. The phrase "the original function is maintained" used for BGL means that a variant of the protein has the allolactose-generating activity and the allolactose-hydrolyzing activity.

The allolactose-generating activity can be measured by, for example, incubating the enzyme with a substrate (i.e. lactose), and measuring the enzyme- and substrate-dependent generation of a product (i.e. allolactose).

The allolactose-hydrolyzing activity can be measured by, for example, incubating the enzyme with a substrate (i.e. allolactose), and measuring the enzyme-and substrate-dependent generation of a product (i.e. glucose or galactose).

The lactose-hydrolyzing activity can be measured by, for example, incubating the enzyme with a substrate (i.e. lactose), and measuring the enzyme- and substrate-dependent generation of a product (i.e. glucose or galactose).

Hereinafter, examples of the conservative variants will be explained.

Homologues of the BGL gene or homologues of BGL can be easily obtained from public databases by, for example, BLAST search or FASTA search using a known nucleotide sequence such as the nucleotide sequence exemplified above or a known amino acid sequence such as the amino acid sequence exemplified above as a query sequence. Furthermore, homologues of the BGL gene can be obtained by, for example, PCR using a chromosome of various organisms as the template, and oligonucleotides prepared on the basis of a known nucleotide sequence such as the nucleotide sequence exemplified above as primers.

The BGL gene may be a gene encoding a protein having a known amino acid sequence such as the amino acid sequence exemplified above, but which includes substitution, deletion, insertion, and/or addition of one or several amino acid residues at one or several positions, so long as the original function is maintained. For example, the N-terminus and/or the C-terminus of the encoded protein may be elongated or shortened. Although the number meant by the term "one or several" mentioned above may differ depending on the positions of amino acid residues in the three-dimensional structure of the protein or the types of amino acid residues, specifically, it may be, for example, 1 to 50, 1 to 40, 1 to 30, 1 to 20, 1 to 10, 1 to 5, or 1 to 3.

The aforementioned substitution, deletion, insertion, and/or addition of one or several amino acid residues are/is a conservative mutation that maintains the original function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, addition, or the like of amino acid residues as mentioned above includes a naturally occurring mutation due to an individual difference, or a difference of species of the organism from which the gene is derived (mutant or variant).

The BGL gene may be a gene encoding a protein having an amino acid sequence showing an identity of, for example, 50% or more, 65% or more, 80% or more, 90% or more, 95% or more, 97% or more, or 99% or more, to the total amino acid sequence of a known amino acid sequence such as the amino acid sequence exemplified above, so long as the original function is maintained.

The BGL gene may also be a gene, such as DNA, that is able to hybridize under stringent conditions with a probe that can be prepared from a known nucleotide sequence such as the nucleotide sequence exemplified above, such as a sequence complementary to a partial or entire sequence of a known nucleotide sequence such as the nucleotide sequence exemplified above, so long as the original function is maintained. The term "stringent conditions" may refer to conditions under which a so-called specific hybrid is formed, and a non-specific hybrid is not formed. Examples of the stringent conditions include those under which highly identical DNAs hybridize to each other, for example, DNAs not less than 50%, 65%, 80%, 90%, 95%, 97%, or 99% identical, hybridize to each other, and DNAs less identical than the above do not hybridize to each other, or conditions of washing of typical Southern hybridization, i.e., conditions of washing once, preferably 2 or 3 times, at a salt concentration and temperature corresponding to 1 × SSC, 0.1% SDS at 60°C, preferably 0.1 × SSC, 0.1% SDS at 60°C, more preferably 0.1 × SSC, 0.1% SDS at 68°C.

The probe used for the aforementioned hybridization may be a part of a sequence that is complementary to the gene as described above. Such a probe can be prepared by PCR using oligonucleotides prepared on the basis of a known nucleotide sequence such as the nucleotide sequence exemplified above as primers and a DNA fragment containing any of the aforementioned genes as a template. As the probe, for example, a DNA fragment having a length of about 300 bp can be used. When a DNA fragment having a length of about 300 bp is used as the probe, the washing conditions of the hybridization may be, for example, 50°C, 2 × SSC and 0.1% SDS.

Furthermore, since the degeneracy of codons differs depending on the host, any codons in the BGL gene may be replaced with respective equivalent codons. That is, the BGL gene may be a variant of the BGL gene exemplified above due to the degeneracy of the genetic code. For example, the BGL gene may be a gene modified so that it has optimal codons according to codon frequencies in a host to be used.

The term "identity" between amino acid sequences means an identity between amino acid sequences calculated by blastp with default scoring parameters (i.e. Matrix, BLOSUM62; Gap Costs, Existence = 11, Extension = 1; Compositional Adjustments, Conditional compositional score matrix adjustment). The term "identity" between nucleotide sequences means an identity between nucleotide sequences calculated by blastn with default scoring parameters (i.e. Match/Mismatch Scores = 1, -2; Gap Costs = Linear).

The aforementioned descriptions concerning conservative variants of the genes and proteins can also be applied *mutatis mutandis* to any genes and proteins other than the BGL gene and BGL.

### <1-3> Methods for increasing activity of protein

Hereinafter, the methods for increasing the activity of a protein will be explained.

The expression "the activity of a protein is increased" may mean that the activity of the protein is increased as compared with a non-modified strain. Specifically, the expression "the activity of a protein is increased" may mean that the activity of the protein per cell is increased as compared with that of a non-modified strain. The term "non-modified strain" used herein may refer to a control strain that has not been modified so that the activity of an objective protein is increased. Examples of the non-modified strain include a wild-type strain and parent strain. Specific examples of the non-modified strain include the respective type strains of the species of microorganisms. Specific examples of the non-modified strain also include strains exemplified above in relation to the description of microorganisms. That is, in an embodiment, the activity of a protein may be increased as compared with a type strain, i.e. the type strain of the species to which the microorganism of the present invention belongs. In another embodiment, the activity of a protein may also be increased as compared with the C. *glutamicum* ATCC 13869 strain. In another embodiment, the activity of a protein may also be increased as compared with the C. *glutamicum* ATCC 13032 strain. In another embodiment, the activity of a protein may also be increased as compared with the *E. coli* K-12 MG1655 strain. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein, but may also mean the transcription amount of a gene (i.e. the amount of mRNA) encoding the protein, or the translation amount of the gene (i.e. the amount of the protein). Furthermore, the state that "the activity of a protein is increased" includes not only a state that the activity of an objective protein is increased in a strain inherently having the activity of the objective protein, but also a state that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.2 times or more, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" may mean that the expression of the gene is increased as compared with a non-modified strain such as a wild-type strain and parent strain. Specifically, the expression "the expression of a gene is increased" may mean that the expression amount of the gene per cell is increased as compared with that of a non-modified strain. More specifically, the expression "the expression of a gene is increased" may mean that the transcription amount of the gene (i.e. the amount of mRNA) is increased, and/or the translation amount of the gene (i.e. the amount of the protein expressed from the gene) is increased. The state that "the expression of a gene is increased" may also be referred to as "the expression of a gene is enhanced". The expression of a gene may be increased to, for example, 1.2 times or more, 1.5 times or more, 2 times or more, or 3 times or more of that of a non-modified strain. Furthermore, the state that "the expression of a gene is increased" includes not only a state that the expression amount of an objective gene is increased in a strain that inherently expresses the objective gene, but also a state that the gene is introduced into a strain that does not inherently express the objective gene, and expressed therein. That is, the phrase "the expression of a gene is increased" may also mean, for example, that an objective gene is introduced into a strain that does not possess the gene, and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination (Miller, J.H., Experiments in Molecular Genetics, 1972, Cold Spring Harbor Laboratory). Examples of the gene transfer method utilizing homologous recombination include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy, or two or more copies of a gene may be introduced. For example, by performing homologous recombination using a sequence which is present in multiple copies on a chromosome as a target, multiple copies of a gene can be introduced into the chromosome. Examples of such a sequence which is present in multiple copies on a chromosome include repetitive DNAs, and inverted repeats located at the both ends of a transposon. Alternatively, homologous recombination may be performed by using an appropriate sequence on a chromosome such as a gene unnecessary for carrying out the present invention as a target. Furthermore, a gene can also be randomly introduced into a chromosome by using a transposon or Mini-Mu (Japanese Patent Laid-open (Kokai) No. 2-109985, U.S. Patent No. 5,882,888, EP805867B1).

Introduction of an objective gene into a chromosome can be confirmed by Southern hybridization using a probe having a sequence complementary to the whole gene or a part thereof, PCR using primers prepared on the basis of the sequence of the gene, or the like.

Furthermore, the copy number of a gene can also be increased by introducing a vector containing the gene into a host. For example, the copy number of an objective gene can be increased by ligating a DNA fragment containing the objective gene with a vector that functions in a host to construct an expression vector of the gene, and transforming the host with the expression vector. The DNA fragment containing the objective gene can be obtained by, for example, PCR using the genomic DNA of a microorganism having the objective gene as the template. As the vector, a vector autonomously replicable in the cell of the host can be used. The vector may be a multi-copy vector. Furthermore, the vector may have a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in *Enterobacteriaceae* bacteria such as *Escherichia coli* include, for example, pUC19, pUC18, pHSG299, pHSG399, pHSG398, pBR322, pSTV29 (all of these are available from Takara Bio), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pCold TF DNA (Takara), pACYC series vectors, and the broad host spectrum vector RSF1010. Specific examples of vector autonomously replicable in coryneform bacteria include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 (Japanese Patent Laid-open (Kokai) No. 3-210184); plasmids pCRY21, pCRY2KE, pCRY2KX, pCRY31, pCRY3KE, and pCRY3KX (Japanese Patent Laid-open (Kokai) No. 2-72876 and U.S. Patent No. 5,185,262); plasmids pCRY2 and pCRY3 (Japanese Patent Laid-open (Kokai) No. 1-191686); pAJ655, pAJ611, and pAJ1844 (Japanese Patent Laid-open (Kokai) No. 58-192900); pCG1 (Japanese Patent Laid-open (Kokai) No. 57-134500); pCG2 (Japanese Patent Laid-open (Kokai) No. 58-35197); pCG4 and pCG11 (Japanese Patent Laid-open (Kokai) No. 57-183799); pPK4 (U.S. Patent No. 6,090,597); pVK4 (Japanese Patent Laid-open (Kokai) No. 9-322774); pVK7 (Japanese Patent Laid-open (Kokai) No. 10-215883); pVK9 (WO2007/046389); pVS7 (WO2013/069634); and pVC7 (Japanese Patent Laid-open (Kokai) No. 9-070291). Specific examples of vector autonomously replicable in coryneform bacteria also include, for example, pVC7H2, which is a variant of pVC7.

When a gene is introduced, it is sufficient that the gene can be expressed by a host. Specifically, it is sufficient that the gene is harbored by a host so that it is expressed under control by a promoter that functions in the host. The term "promoter that functions in a host" may refer to a promoter that shows a promoter activity in the host. The promoter may be a promoter derived from the host, or a heterogenous promoter. The promoter may be the native promoter of the gene to be introduced, or a promoter of another gene. As the promoter, for example, such a stronger promoter as described herein may also be used.

A terminator for termination of gene transcription may be located downstream of the gene. The terminator is not particularly limited so long as it functions in the host. The terminator may be a terminator derived from the host, or a heterogenous terminator. The terminator may be the native terminator of the gene to be introduced, or a terminator of another gene. Specific examples of the terminator include, for example, T7 terminator, T4 terminator, fd phage terminator, *tet* terminator, and *trpA* terminator.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more of genes are introduced, it is sufficient that the genes each are expressibly harbored by the host. For example, all the genes may be carried by a single expression vector or a chromosome. Furthermore, the genes may be separately carried by two or more expression vectors, or separately carried by a single or two or more expression vectors and a chromosome. An operon constituted by two or more genes may also be introduced. When "introducing two or more genes", for example, respective genes encoding two or more kinds of proteins (such as enzymes), respective genes encoding two or more subunits constituting a single protein complex (such as enzyme complex), or a combination of these genes may be introduced.

The gene to be introduced is not particularly limited so long as it encodes a protein that functions in the host. The gene to be introduced may be a gene derived from the host, or may be a heterogenous gene. The gene to be introduced can be obtained by, for example, PCR using primers designed on the basis of the nucleotide sequence of the gene, and using the genomic DNA of an organism having the gene, a plasmid carrying the gene, or the like as a template. The gene to be introduced may also be totally synthesized, for example, on the basis of the nucleotide sequence of the gene (Gene, 60(1), 115-127 (1987)). The obtained gene can be used as it is, or after being modified as required. That is, a gene can be modified to obtain a variant thereof. A gene can be modified by a known technique. For example, an objective mutation can be introduced into an objective site of DNA by the site-specific mutation method. That is, the coding region of a gene can be modified by the site-specific mutation method so that a specific site of the encoded protein include substitution, deletion, insertion, and/or addition of amino acid residues. Examples of the site-specific mutation method include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of a gene may be totally synthesized.

Incidentally, when a protein functions as a complex consisting of a plurality of subunits, a part or all of these subunits may be modified, so long as the activity of the protein is eventually increased. That is, for example, when the activity of a protein is increased by increasing the expression of a gene, the expression of a part or all of these genes that encode the respective subunits may be enhanced. It is usually preferable to enhance the expression of all of those genes encoding the subunits. Furthermore, the subunits constituting the complex may be derived from one kind of organism or two or more kinds of organisms, so long as the complex has a function of the objective protein. That is, for example, genes of the same organism encoding a plurality of subunits may be introduced into a host, or genes of different organisms encoding a plurality of subunits may be introduced into a host.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The term "expression control sequence" may collectively refer to sites that affect the expression of a gene. Examples of the expression control sequence include, for example, promoter, Shine-Dalgarno (SD) sequence (also referred to as ribosome binding site (RBS)), and spacer region between RBS and the start codon. Expression control sequences can be identified by using a promoter search vector or gene analysis software such as GENETYX. These expression control sequences can be modified by, for example, a method of using a temperature sensitive vector, or the Red driven integration method (WO2005/010175).

The transcription efficiency of a gene can be improved by, for example, replacing the promoter of the gene on a chromosome with a stronger promoter. The term "stronger promoter" may refer to a promoter providing an improved transcription of a gene compared with an inherently existing wild-type promoter of the gene. Examples of stronger promoters include, for example, the known high expression promoters such as T7 promoter, *trp* promoter, *lac* promoter, *thr* promoter, *tac* promoter, *trc* promoter, *tet* promoter, *araBAD* promoter, *rpoH* promoter, *msrA* promoter, Pm1 promoter (derived from the genus *Bifidobacterium*)*,* PR promoter, and PL promoter. Furthermore, as the stronger promoter, a highly-active type of an existing promoter may also be obtained by using various reporter genes. Examples of stronger promoters usable in coryneform bacteria include, for example, the artificially modified P54-6 promoter (Appl. Microbiol. Biotechnol., 53, 674-679 (2000)), *pta, aceA, aceB, adh,* and *amyE* promoters inducible in coryneform bacteria with acetic acid, ethanol, pyruvic acid, or the like, *cspB, SOD,* and *tuf* (EF-Tu) promoters, which are potent promoters capable of providing a large expression amount in coryneform bacteria (Journal of Biotechnology, 104 (2003) 311-323; Appl. Environ. Microbiol., 2005 Dec; 71 (12):8587-96), as well as P2 promoter (WO2018/079684), P3 promoter (WO2018/079684), *lac* promoter, *tac* promoter, *trc* promoter, and F1 promoter. For example, by making the -35 and -10 regions in a promoter region closer to the consensus sequence, the activity of the promoter can be enhanced (WO00/18935). Examples of highly active-type promoter include various tac-like promoters (Katashkina JI et al., Russian Federation Patent Application No. 2006134574). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" may refer to a SD sequence that provides an improved translation of mRNA compared with the inherently existing wild-type SD sequence of the gene. Examples of stronger SD sequences include, for example, RBS of the gene *10* derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon more frequently used. That is, the gene to be introduced may be modified, for example, so as to contain optimal codons according to the frequencies of codons observed in a host to be used. Codons can be replaced by, for example, the site-specific mutation method for introducing an objective mutation into an objective site of DNA. Alternatively, a gene fragment in which objective codons are replaced may be totally synthesized. Frequencies of codons in various organisms are disclosed in the "Codon Usage Database" (http://www.kazusa.or.jp/codon; Nakamura, Y. et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any appropriate combination.

Furthermore, the modification that increases the activity of a protein can also be attained by, for example, enhancing the specific activity of the protein. Enhancement of the specific activity may also include desensitization to feedback inhibition. A protein showing an enhanced specific activity can be obtained by, for example, searching various organisms. Furthermore, a highly-active type of an existing protein may also be obtained by introducing a mutation into the existing protein. The mutation to be introduced may be, for example, substitution, deletion, insertion, and/or addition of one or several amino acid residues at one or several position of the protein. The mutation can be introduced by, for example, such a site-specific mutation method as mentioned above. The mutation may also be introduced by, for example, a mutagenesis treatment. Examples of the mutagenesis treatment include irradiation of X-ray, irradiation of ultraviolet, and a treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used, or may be used in any appropriate combination with such methods for enhancing gene expression as mentioned above.

The method for the transformation is not particularly limited, and conventionally known methods can be used. There can be used, for example, a method of treating recipient cells with calcium chloride so as to increase the permeability thereof for DNA, which has been reported for the *Escherichia coli* K-12 strain (Mandel, M. and Higa, A., J. Mol. Biol., 1970, 53, 159-162), and a method of preparing competent cells from cells which are in the growth phase, followed by transformation with DNA, which has been reported for *Bacillus subtilis* (Duncan, C.H., Wilson, G.A. and Young, F.E., Gene, 1977, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, the electric pulse method reported for coryneform bacteria (Japanese Patent Laid-open (Kokai) No. 2-207791) can also be used.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wild-type strain or parent strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be increased to, for example, 1.2 times or more, 1.5 times or more, 2 times or more, or 3 times or more of that of a non-modified strain.

An increase in the amount of a protein can be confirmed by Western blotting using antibodies (Sambrook, J., et al., Molecular Cloning A Laboratory Manual/Third Edition, Cold Spring Harbor Laboratory Press, Cold Spring Harbor (USA), 2001). The amount of the protein (such as the number of molecules of the protein per cell) may be increased to, for example, 1.2 times or more, 1.5 times or more, 2 times or more, or 3 times or more of that of a non-modified strain.

The aforementioned methods for increasing the activity of a protein can be used for enhancement of the activities of any proteins and enhancement of the expression of any genes.

### <1-4> Production of the cells of the present invention

The cells of the present invention can be produced by culturing the microorganism of the present invention in a culture medium.

The culture conditions are not particularly limited, so long as the microorganism of the present invention can proliferate, and a functional BGL is expressed. The culture conditions may be appropriately set according to various conditions such as the type of microorganism.

As the culture medium, for example, a usual culture medium used for culture of bacteria such as *Escherichia coli* can be used as it is, or after appropriate modification. As the culture medium, for example, a liquid culture medium containing a carbon source, nitrogen source, phosphorus source, and sulfur source, as well as component(s) selected from other various organic components and inorganic components as required can be used. Types and concentrations of the culture medium components can be appropriately set by a skilled artisan.

Specific examples of the carbon source include, for example, saccharides such as glucose, fructose, sucrose, lactose, galactose, xylose, arabinose, blackstrap molasses, hydrolysates of starches, and hydrolysates of biomass, organic acids such as acetic acid, fumaric acid, citric acid, succinic acid, and malic acid, alcohols such as glycerol, crude glycerol, and ethanol, and aliphatic acids. As the carbon source, one kind of carbon source may be used, or two or more kinds of carbon sources may be used in combination.

Specific examples of the nitrogen source include, for example, ammonium salts such as ammonium sulfate, ammonium chloride, and ammonium phosphate, organic nitrogen sources such as peptone, yeast extract, meat extract, and soybean protein decomposition products, ammonia, and urea. As the nitrogen source, one kind of nitrogen source may be used, or two or more kinds of nitrogen sources may be used in combination.

Specific examples of the phosphate source include, for example, phosphoric acid salts such as potassium dihydrogenphosphate and dipotassium hydrogenphosphate, and phosphoric acid polymers such as pyrophosphoric acid. As the phosphate source, one kind of phosphate source may be used, or two or more kinds of phosphate sources may be used in combination.

Specific examples of the sulfur source include, for example, inorganic sulfur compounds such as sulfates, thiosulfates, and sulfites, and sulfur-containing amino acids such as cysteine, cystine, and glutathione. As the sulfur source, one kind of sulfur source may be used, or two or more kinds of sulfur sources may be used in combination.

Specific examples of other various organic components and inorganic components include, for example, inorganic salts such as sodium chloride and potassium chloride; trace metals such as iron, manganese, magnesium, and calcium; vitamins such as vitamin B1, vitamin B2, vitamin B6, nicotinic acid, nicotinamide, and vitamin B12; amino acids; nucleic acids; and organic components containing those such as peptone, casamino acid, yeast extract, and soybean protein decomposition product. As other various organic components and inorganic components, one kind of component may be used, or two or more kinds of components may be used in combination.

Furthermore, when an auxotrophic strain that requires a nutrient such as an amino acid for growth thereof is used, it is preferable to supply a required nutrient to the culture medium. Furthermore, when a gene was introduced using a vector carrying an antibiotic resistant gene, it is preferable to supply a corresponding antibiotic to the culture medium.

The culture can be performed, for example, aerobically as a culture with aeration or shaking. The oxygen concentration may be controlled to be, for example, 5 to 50% of the saturated dissolved oxygen concentration, or preferably 20 to 40% of the saturated dissolved oxygen concentration. The culture temperature may be, for example, 20 to 45°C, 25 to 40°C, or 30 to 37°C. The pH upon culturing may be, for example, 5 to 9. For adjusting pH, organic or inorganic alkaline or acidic substances such as, for example, calcium carbonate, ammonia gas, and aqueous ammonia can be used. The culture can be performed as batch culture, fed-batch culture, continuous culture, or a combination of these. The culture may also be performed separately as seed culture and main culture. In such a case, the culture conditions of the seed culture and the main culture may be or may not be the same. The seed culture may be performed, for example, by using a plate culture medium or a liquid culture medium. The culture can be performed, for example, until the microorganism of the present invention proliferates to desired degree. The culture period may be, for example, 10 to 120 hours. The culture may be continued, for example, until the carbon source present in the culture medium is consumed, or until the activity of the microorganism of the present invention is lost.

Upon culturing, expression of the BGL gene may be induced as required. Conditions for expression induction may be appropriately chosen according to various conditions such as the type of promoter.

By culturing the microorganism of the present invention as described above, cells having BGL (i.e. the cells of the present invention) are generated, and thereby a culture broth containing the cells is obtained.

The cells may be used for the conversion reaction while being present in the culture broth (specifically, culture medium), or after being collected from the culture broth (specifically, culture medium). The method for collecting the cells from the culture medium is not particularly limited, and for example, known methods can be used. Examples of such methods can include, for example, spontaneous precipitation, centrifugation, and filtration. A flocculant may also be used. These methods may be independently used, or may be used in an appropriate combination. The collected cells may be washed as required by using an appropriate medium. The collected cells may be re-suspended as required by using an appropriate medium. Examples of the medium usable for washing or suspending the cells can include, for example, aqueous media (aqueous solvents) such as water and aqueous buffer. That is, the cells may be used for the conversion reaction, for example, in the form of being isolated to desired degree, or in the form of being contained in a material such as a culture broth or a re-suspension. That is, examples of the cells of the present invention include cells collected from a culture broth, and a material containing cells such as a culture broth or a re-suspension. In other words, examples of the cells of the present invention include cells collected from a culture broth, and cells contained in a material such as a culture broth or a re-suspension. The cells or a fraction containing the same may also be used for the conversion reaction after being subjected to a treatment, such as dilution, concentration, and pH adjustment, as required. The cells may also be used for the conversion reaction, for example, in the form of immobilized cells, which are immobilized on a carrier such as acrylamide and carrageenan.

The state of cells used for the conversion reaction is not particularly limited, so long as the cells have a functional BGL. The cells may be viable cells or dead cells. The cells may be used in the form of containing viable cells. That is, the cells, for example, may consist of viable cells, or may be a mixture of viable cells and dead cells. The ratio of viable cells in the cells may be, for example, 10% or more, 30% or more, 50% or more, 70% or more, or 90% or more, based on the number of cells. The number of viable cells can be measured as colony forming unit (cfu). In particular, the cells may be used for the conversion reaction without being subjected to a treatment for increasing membrane permeability. That is, the cells of the present invention may be, in particular, cells not subject to a treatment for increasing membrane permeability. The cells not subject to a treatment for increasing membrane permeability are also referred to as "untreated cells". That is, in the present invention, allolactose can be produced even by using the untreated cells. Specifically, for example, allolactose can be produced by carrying out the conversion reaction using the untreated cells under high osmotic conditions. Examples of the treatment for increasing membrane permeability include drying treatment, freeze-thaw treatment, surfactant treatment, and organic solvent treatment. That is, Examples of the untreated cells include cells not subject to any of drying treatment, freeze-thaw treatment, surfactant treatment, and organic solvent treatment.

### <2> Method for producing allolactose

Allolactose can be produced by carrying out the conversion reaction using the cells of the present invention and lactose.

The conversion reaction can be carried out in an appropriate liquid. The liquid in which the conversion reaction is carried out is also referred to as a "reaction mixture". Specifically, the conversion reaction can be carried out by allowing the cells of the present invention and lactose to coexist in an appropriate reaction mixture. The conversion reaction, for example, may be carried out by a batch method or may be carried out by a column method. In the case of the batch method, the conversion reaction can be carried out by, for example, mixing the cells of the present invention and lactose in a reaction mixture contained in a reaction vessel. The conversion reaction may be carried out statically, or may be carried out with stirring or shaking the reaction mixture. In the case of the column method, the conversion reaction can be carried out by, for example, passing a reaction mixture containing lactose through a column filled with immobilized cells. Examples of the reaction mixture can include those based on an aqueous medium (aqueous solvent) such as water and aqueous buffer.

The conversion reaction is carried out in the presence of the substance X (i.e. a substance that inhibits hydrolysis of allolactose). That is, the reaction mixture further contains the substance X. In the conversion reaction, hydrolysis of allolactose may be inhibited by the substance X, and thereby allolactose may be efficiently generated and accumulated. Hydrolysis of allolactose may be completely inhibited or partially inhibited. Hydrolysis of allolactose to be inhibited by the substance X may be hydrolysis catalyzed by the microorganism of the present invention (i.e. by the cells of the present invention). Hydrolysis of allolactose to be inhibited by the substance X may specifically be hydrolysis catalyzed by BGL possessed by the microorganism of the present invention (i.e. by BGL possessed by the cells of the present invention). Hydrolysis of allolactose may be inhibited by, for example, product inhibition. That is, Examples of the substance X include hydrolysis products of allolactose. Examples of the hydrolysis products of allolactose include glucose and galactose. Particular examples of the hydrolysis products of allolactose include glucose. The phrase "hydrolysis product of allolactose" as the substance X refers to a component that can be generated by hydrolysis of allolactose, regardless of whether the substance was actually generated by hydrolysis of allolactose. That is, the "hydrolysis product of allolactose" as the substance X, such as glucose and galactose, may be one obtained by any means. As the substance X, one kind of substance may be used, or two or more kinds of substances may be used in combination.

The substance X may also function as a substance that inhibits hydrolysis of lactose. That is, in the conversion reaction, hydrolysis of lactose may be inhibited by the substance X, and thereby allolactose may be efficiently generated and accumulated. Hydrolysis of lactose may be completely inhibited or partially inhibited. Specifically, for example, lactose may be used for production of allolactose by inhibiting hydrolysis of lactose, and thereby allolactose may be efficiently generated and accumulated. Hydrolysis of lactose to be inhibited by the substance X may be hydrolysis catalyzed by the microorganism of the present invention (i.e. by the cells of the present invention). Hydrolysis of lactose to be inhibited by the substance X may specifically be hydrolysis catalyzed by BGL possessed by the microorganism of the present invention (i.e. by BGL possessed by the cells of the present invention). Hydrolysis of lactose may be inhibited by, for example, product inhibition. For example, the hydrolysis product of allolactose is also a hydrolysis product of lactose. Hence, the hydrolysis product of allolactose (i.e. the hydrolysis product of lactose) may inhibit hydrolysis of lactose by product inhibition.

In cases where the substance X is generated from lactose during the conversion reaction (including cases where the substance X is generated from lactose via other component(s) such as allolactose), cases where the conversion reaction is carried out in the presence of only the thus-generated substance X shall not fall within the case where "the conversion reaction is carried out in the presence of the substance X". In other words, the phrase "the conversion reaction is carried out in the presence of the substance X" means that the conversion reaction is carried out at least in the presence of the substance X that was externally supplied.

The reaction mixture may further contain component(s) other than lactose, the substance X, and the cells of the present invention. The component other than lactose, the substance X, and the cells of the present invention is also referred to as "other component". The other component is not particularly limited, so long as allolactose is generated to desired degree. Examples of the other component include pH buffers and culture medium components.

The conditions for the conversion reaction, such as the concentrations of components, reaction time, reaction temperature, and pH of the reaction mixture, are not particularly limited, so long as allolactose is generated to desired degree.

The reaction conditions may be constant from the start to the end of the conversion reaction, or they may change during the conversion reaction. The phrase "the reaction conditions change during the conversion reaction" includes not only cases where the reaction conditions temporally change, but also includes cases where the reaction conditions spatially change. The phrase "the reaction conditions spatially change" means that, for example, when the conversion reaction is carried out by the column method, the reaction conditions differ depending on position in the flow.

The concentration of lactose may be, for example, a high concentration. The concentration of lactose, for example, may be 120 g/L or more, 130 g/L or more, 140 g/L or more, 150 g/L or more, 160 g/L or more, 170 g/L or more, 180 g/L or more, 190 g/L or more, 200 g/L or more, 220 g/L or more, or 250 g/L or more, or may be the saturated concentration or less, 400 g/L or less, 350 g/L or less, 300 g/L or less, 250 g/L or less, 220 g/L or less, or 200 g/L or less, or may be within a range defined by a non-contradictory combination thereof. The concentration of lactose may be, specifically, for example, 120 g/L to the saturated concentration, 150 g/L to the saturated concentration, or 180 g/L to the saturated concentration. The concentration of lactose may also be, specifically, for example, 120 g/L to 400 g/L, 150 g/L to 300 g/L, 180 g/L to 250 g/L. Lactose that cannot be dissolved (e.g. lactose in excess of the saturated concentration) may or may not be present in the reaction system.

The concentration of the substance X may be, for example, a high concentration. The concentration of the substance X, for example, may be 120 g/L or more, 130 g/L or more, 140 g/L or more, 150 g/L or more, 160 g/L or more, 170 g/L or more, 180 g/L or more, 190 g/L or more, 200 g/L or more, 220 g/L or more, or 250 g/L or more, or may be the saturated concentration or less, 400 g/L or less, 350 g/L or less, 300 g/L or less, 250 g/L or less, 220 g/L or less, or 200 g/L or less, or may be within a range defined by a non-contradictory combination thereof. The concentration of the substance X may be, specifically, for example, 120 g/L to the saturated concentration, 150 g/L to the saturated concentration, or 180 g/L to the saturated concentration. The concentration of the substance X may also be, specifically, for example, 120 g/L to 400 g/L, 150 g/L to 300 g/L, 180 g/L to 250 g/L. When the substance X consists of two or more components, the phrase "concentration of the substance X" refers to the total concentration of the substance X. The substance X that cannot be dissolved (e.g. the substance X in excess of the saturated concentration) may or may not be present in the reaction system.

The concentration of the cells of the present invention, for example, may be 1 or more, 2 or more, 3 or more, 4 or more, 5 or more, 7 or more, 10 or more, 15 or more, or 20 or more, or may be 300 or less, 200 or less, 150 or less, 100 or less, 70 or less, 50 or less, 30 or less, 20 or less, 10 or less, 5 or less, 4 or less, 3 or less, or 2 or less, or may be within a range defined by a non-contradictory combination thereof, in term of the optical density (OD) at 600 nm. The concentration of the cells of the present invention may be, specifically, for example, 1 to 300, 2 to 100, or 3 to 20, in term of the optical density (OD) at 600 nm.

The conversion reaction may be carried out under high osmotic conditions. By carrying out the conversion reaction under high osmotic conditions, for example, intracellular component(s) such as BGL may be eluted from the cells, and thereby allolactose may be efficiently generated and accumulated. The phrase "high osmotic conditions" may refer to, for example, conditions where the solute concentration in the reaction mixture is 1 Eq/L or more, 1.1 Eq/L or more, 1.2 Eq/L or more, 1.3 Eq/L or more, 1.4 Eq/L or more, 1.5 Eq/L or more, 1.6 Eq/L or more, 1.7 Eq/L or more, 1.8 Eq/L or more, 1.9 Eq/L or more, or 2 Eq/L or more. The solute concentration in the reaction mixture may also be, for example, 4 Eq/L or less, 3 Eq/L or less, 2.5 Eq/L or less, 2.2 Eq/L or less, 2 Eq/L or less, 1.9 Eq/L or less, 1.8 Eq/L or less, 1.7 Eq/L or less, 1.6 Eq/L or less, or 1.5 Eq/L or less. The solute concentration in the reaction mixture may also be, for example, within a range defined by a non-contradictory combination of the ranges exemplified above. The solute concentration in the reaction mixture may also be, specifically, for example, 1 to 4 Eq/L, 1.3 to 3 Eq/L, or 1.5 to 2 Eq/L. The phrase "solute" referred to herein refers to the components dissolved in the reaction mixture. That is, for example, the total concentration of the solute (i.e. the total concentration of all the components dissolved in the reaction mixture) may be set to the solute concentration exemplified above. In other words, the phrase "high osmotic conditions" may also refer to, for example, conditions where the total concentration of the solute in the reaction mixture is the solute concentration exemplified above. Examples of the solute include lactose and the substance X. That is, for example, the total concentration of lactose and the substance X may be set to the solute concentration exemplified above. In other words, the phrase "high osmotic conditions" may also refer to, for example, conditions where the total concentration of lactose and the substance X in the reaction mixture is the solute concentration exemplified above. When the solute is a substance that does not ionize, the phrase "Eq/L" may be read as "M". For example, lactose and such a substance X as exemplified above (e.g. the hydrolysis products of allolactose such as glucose and galactose) may each be regarded as a substance that does not ionize. Hence, for example, when the total concentration of lactose and such a substance X as exemplified above is set to the solute concentration exemplified above, the phrase "Eq/L" may be read as "M".

Each component may or may not be contained in the reaction mixture at the concentration exemplified above over the whole period of the conversion reaction. Each component may be contained in the reaction mixture at the concentration exemplified above, for example, at the start of the conversion reaction. That is, the phase "the concentration of a certain component in the conversion reaction is within a certain range" means that there is a period where the conversion reaction is carried out with the component at a concentration within the range, and may specifically mean that the conversion reaction is started with the component at a concentration within the range. The phrase "at the start of a reaction" may refer to the time when lactose, the substance X, and the cells of the present invention come to coexist in the reaction system at a predetermined concentration. The phrase "at the start of a reaction" may refer to the time when lactose, the substance X, and the cells of the present invention come to coexist in the reaction system at the concentration exemplified above. The concentration of each component may change, for example, during the conversion reaction. For example, the concentration of lactose may decrease during the conversion step. Specifically, for example, lactose may be consumed during the conversion step, and thereby the concentration of lactose may decrease. Also, for example, the concentration of the cells of the present invention may decrease during the conversion step. Specifically, for example, the cells of the present invention may lyse during the conversion step, and thereby the concentration of the cells of the present invention may decrease. Also, for example, the concentration of the substance X may increase during the conversion step. Specifically, for example, lactose and/or allolactose may be hydrolyzed during the conversion step, and thereby the concentration of the substance X may increase.

Component(s) selected from lactose, the substance X, the cells of the present invention, and other components may be supplied to the reaction mixture independently, or in an appropriate combination. For example, lactose may be additionally supplied to the reaction mixture dependently on consumption of lactose. Any component may be supplied once or more times, or may be supplied continuously.

The reaction time, for example, may be 12 hours or more, 1 day or more, 2 days or more, 3 days or more, 4 days or more, 5 days or more, 7 days or more, 10 days or more, 15 days or more, or 20 days or more, or may be 210 days or less, 180 days or less, 150 days or less, 120 days or less, 90 days or less, 60 days or less, 40 days or less, 30 days or less, 20 days or less, 15 days or less, 10 days or less, 7 days or less, 5 days or less, 4 days or less, or 3 days or less, or may be within a range defined by a non-contradictory combination thereof. The reaction time may be, specifically, for example, 12 hours to 20 days, 1 day to 15 days, or 3 days to 10 days. In the case of the column method, the flow rate of the reaction mixture may be, for example, such a flow rate that the reaction time is within the range of the reaction time exemplified above.

The conversion reaction may be carried out, for example, until allolactose is generated and accumulated to desired degree. The accumulation amount of allolactose may be, for example, 1 g/L or more, 2 g/L or more, 5 g/L or more, 10 g/L or more, 15 g/L or more, 20 g/L or more, 25 g/L or more, 30 g/L or more, 35 g/L or more, 40 g/L or more, 45 g/L or more, or 50 g/L or more.

The reaction temperature, for example, may be 10°C or more, 15°C or more, 20°C or more, or 30°C or more, may be 35°C or less, 30°C or less, 25°C or less, 20°C or less, or 15°C or less, or may be within a range defined by a non-contradictory combination thereof. The reaction temperature may be, in particular, 30°C or less. The reaction temperature may be, specifically, for example, 10 to 30°C, 15 to 30°C, or 20 to 30°C. The reaction temperature may or may not be regulated. The conversion reaction may be carried out, for example, at a room temperature (e.g. 25°C). The reaction temperature may typically be within the range of the temperature exemplified above over the whole period of the conversion reaction, but it may temporally be out of that range. That is, the phrase "the temperature in the conversion reaction is within a certain range" or "the conversion reaction is carried out at a certain range of temperature" include not only cases where the temperature is within the range over the whole period of the conversion reaction, but also includes cases where the temperature is temporally be out of the range. The length of "temporally" is not particularly limited, so long as allolactose is generated to desired degree. The phrase "temporally" may refer to, for example, a period having a length of 20% or less, 15% or less, 10% or less, 5% or less, 3% or less, or 1% or less of the whole period of the conversion reaction.

The reaction pH, for example, may be 4 or more, 5 or more, 6 or more, 7 or more, or 8 or more, or may be 10 or less, 9 or less, 8 or less, 7 or less, or 6 or less, or may be within a range defined by a non-contradictory combination thereof. The reaction pH may be, specifically, for example, 6 to 8. The reaction pH may or may not be regulated. The reaction pH may typically be within the range of the pH exemplified above over the whole period of the conversion reaction, but it may temporally be out of that range. That is, the phrase "the pH in the conversion reaction is within a certain range" or "the conversion reaction is carried out at a certain range of pH" include not only cases where the pH is within the range over the whole period of the conversion reaction, but also includes cases where the pH is temporally be out of the range. The length of "temporally" is not particularly limited, so long as allolactose is generated to desired degree. The phrase "temporally" may refer to, for example, a period having a length of 20% or less, 15% or less, 10% or less, 5% or less, 3% or less, or 1% or less of the whole period of the conversion reaction.

By carrying out the conversion reaction as described above, a reaction mixture containing allolactose is obtained.

Production of allolactose can be confirmed by known methods used for detection or identification of compounds. Examples of such methods include, for example, HPLC, UPLC, LC/MS, GC/MS, and NMR. These methods can be independently used, or can be used in an appropriate combination.

The reaction mixture containing allolactose can be used as it is, or after being subject to a treatment, such as concentration, dilution, heating, and cell removal, as required.

Furthermore, generated allolactose can be collected from the reaction mixture as required. That is, the method of the present invention may comprise collecting generated allolactose from the reaction mixture. Collection of allolactose generated can be carried out by known methods used for separation and purification of compounds. Examples of such methods include, for example, ion-exchange resin method, precipitation method, membrane treatment method, and crystallization method. These methods can be used independently or in an appropriate combination. Purification of allolactose can be carried out to desired degree. When allolactose is precipitated in the reaction mixture, it can be collected by centrifugation, filtration, or the like. Allolactose precipitated in the reaction mixture may also be isolated together with allolactose dissolving in the reaction mixture, after allolactose dissolving in the reaction mixture is crystallized.

Collected allolactose may contain component(s) other than allolactose in addition to allolactose. Examples of the component other than allolactose include the cells, the reaction mixture components, and moisture. The purity of collected allolactose may be, for example, 30%(w/w) or higher, 50%(w/w) or higher, 70%(w/w) or higher, 80%(w/w) or higher, 90%(w/w) or higher, or 95%(w/w) or higher.

### <3> Use of allolactose

The use purpose of produced allolactose is not particularly limited. Allolactose can be used, for example, for inducing gene expression. That is, allolactose can be used as a gene expression inducer. That is, the method for producing allolactose may also be a method for producing a gene expression inducer.

Allolactose can be used, specifically, for example, induced expression of a gene under control of an allolactose-inducible promoter. The phrase "allolactose-inducible promoter" refers to a promoter inducibly expressing a gene ligated immediately downstream of the promoter in the presence of allolactose. The phrase "in the presence of allolactose" may specifically mean that allolactose is present in a culture medium. The phrase "a gene is inducibly expressed in the presence of allolactose" may specifically mean, for example, that the expression amount of a gene in the presence of allolactose is 2 times or more, 3 times or more, or 4 times or more of the expression amount of the gene in the absence of allolactose. The phrase "a gene is inducibly expressed in the presence of allolactose" also includes cases where a gene is expressed in the presence of allolactose whereas the gene is not expressed in the absence of allolactose. The gene to be inducibly expressed under control of an allolactose-inducible promoter is also referred to as "objective gene".

The phrase "an objective gene is expressed under control of an allolactose-inducible promoter" includes not only cases where the objective gene is expressed directly from the allolactose-inducible promoter (direct expression), but also includes cases where the expression of the objective gene is indirectly induced via the expression of another gene (a gene other than the objective gene) from the allolactose-inducible promoter (indirect expression). In the case of the direct expression, for example, when the objective gene is ligated downstream of the allolactose-inducible promoter and the expression of the gene from the allolactose-inducible promoter is induced, the gene thereby can be expressed directly from the allolactose-inducible promoter. In the case of the indirect expression, for example, the expression of the objective gene from another promoter (a promoter other than the allolactose-inducible promoter) can be indirectly induced via a product (such as a transcription product or a translation product) of another gene expressed directly from the allolactose-inducible promoter. For example, transcription of a gene from a T3 promoter, T5 promoter, T7 promoter, and SP6 promoter was performed by a T3 RNA polymerase, T5 RNA polymerase, T7 RNA polymerase, and SP6 RNA polymerase, respectively. Hence, for example, when the objective gene is ligated downstream of the T3 promoter, T5 promoter, T7 promoter, or SP6 promoter and the corresponding RNA polymerase is inducibly expressed from the allolactose-inducible promoter, expression of the objective gene thereby can be indirectly induced. Induction of expression of a gene from the allolactose-inducible promoter is also referred to as "induction of an allolactose-inducible promoter".

Examples of the allolactose-inducible promoter include lac promoter and tac promoter. That is, the allolactose-inducible promoter may be, for example, a promoter having a known nucleotide sequence of lac promoter or tac promoter. The allolactose-inducible promoter may be, for example, a conservative variant of the allolactose-inducible promoter exemplified above, e.g. a conservative variant of the promoter having a known nucleotide sequence of lac promoter or tac promoter. That is, the allolactose-inducible promoter exemplified above, e.g. a conservative variant of the promoter having a known nucleotide sequence of lac promoter or tac promoter, can be used as it is, or after being modified as required. The phrase "lac promoter" includes not only the lac promoter exemplified above, e.g. the promoter having a known nucleotide sequence of lac promoter, but also includes conservative variants thereof. The phrase "tac promoter" includes not only the tac promoter exemplified above, e.g. the promoter having a known nucleotide sequence of tac promoter, but also includes conservative variants thereof. The aforementioned descriptions concerning conservative variants of BGL can be applied *mutatis mutandis* to conservative variants of the allolactose-inducible promoter. For example, the allolactose-inducible promoter may be a DNA having a nucleotide sequence showing a homology of 80% or higher, preferably 90% or higher, more preferably 95% or higher, still more preferably 97% or higher, particularly preferably 99% or higher, to a known nucleotide sequence of lac promoter or tac promoter, so long as the original function is maintained. Specific examples of variants of lac promoter include lacUV5 promoter. Specific examples of variants of tac promoter include various tac-like promoters (Katashkina JI et al., Russian Federation Patent Application No. 2006134574). The phrase "original function" used for the allolactose-inducible promoter refers to a function of inducibly expressing a gene ligated immediately downstream of the promoter in the presence of allolactose. The function of the allolactose-inducible promoter can be confirmed by, for example, confirming an induced expression of a gene due to supply of allolactose to a culture medium. The induced expression of a gene can be confirmed by, for example, using a reporter gene. Similarly, when using the allolactose-inducible promoter in combination with another promoter such as the T3 promoter, such another promoter can also be used as it is, or after being modified as required.

The type of the objective gene is not particularly limited. Examples of the objective gene include genes effective for production of an objective substance. The type of the objective substance is not particularly limited. Examples of the objective substance include SAM-dependent metabolites, aldehydes, L-amino acids, nucleotides, organic acids, gamma-glutamyl peptides, sphingoids, proteins, and RNAs. Examples of the genes effective for production of the objective substance include genes encoding proteins whose increased activity is effective for production of the objective substance. Examples of such proteins include biosynthetic enzymes of the objective substance. Furthermore, when the objective substance is an expression product of a gene (e.g. protein or RNA), examples of the genes effective for production of the objective substance include genes encoding the objective substance.

Expression of the objective gene can be induced, for example, during cultivation of a microorganism having the objective gene. That is, the present invention provides, for example, a method for inducing expression of an objective gene, the method comprising a step of culturing a microorganism having the objective gene in a culture medium containing allolactose, wherein the objective gene is expressed under control of an allolactose-inducible promoter. The aforementioned descriptions concerning cultivation of the microorganism of the present invention can be applied *mutatis mutandis* to cultivation of the microorganism having the objective gene, provided that the objective gene is inducibly expressed by allolactose. The microorganism having the objective gene may or may not be a microorganism inherently having the objective gene that is expressed under control of an allolactose-inducible promoter. The microorganism having the objective gene may be, for example, a microorganism inherently not having the objective gene that is expressed under control of an allolactose-inducible promoter but modified so as to have the objective gene that is expressed under control of an allolactose-inducible promoter. To the microorganism, the aforementioned descriptions concerning the microorganism in explanation of the microorganism of the present invention can be applied *mutatis mutandis.* To modification of the microorganism, the aforementioned descriptions concerning modification of the microorganism in explanation of the microorganism of the present invention can be applied *mutatis mutandis.*

Allolactose may be present in the culture medium over the whole period of the culture, or may be present in the culture medium during only a partial period of the culture. That is, the phrase "culturing a microorganism in a culture medium containing allolactose" does not necessarily mean that allolactose is present in the culture medium over the whole period of the culture. For example, allolactose may be or may not be present in the culture medium from the start of the culture. When allolactose is not present in the culture medium at the time of the start of the culture, allolactose is supplied to the culture medium after the start of the culture. Timing of the supply can be appropriately determined according to various conditions such as the length of the culture period. For example, after the cells proliferates to desired degree, allolactose may be supplied to the culture medium. The concentration of allolactose in the culture medium is not particularly limited, so long as expression of the objective gene is induced. The concentration of allolactose in the culture medium, for example, may be 0.005 mM or more, 0.01 mM or more, 0.05 mM or more, 0.1 mM or more, 0.5 mM or more, or 1 mM or more, or may be 10 mM or less, 5 mM or less, 2 mM or less, 1 mM or less, 0.5 mM or less, or 0.1 mM or less, or may be within a range defined by a non-contradictory combination thereof. The concentration of allolactose in the culture medium may be, specifically, for example, 0.005 to 10 mM, 0.01 to 5 mM, or 0.05 to 2 mM. Allolactose may or may not be present in the culture medium at a concentration within the range exemplified above over the whole period of the culture. For example, allolactose may be present in the culture medium at a concentration within the range exemplified above at the start of the culture, or it may be supplied to the culture medium so that a concentration within the range exemplified above is attained after the start of the culture.

The microorganism having the objective gene may have any characteristics, so long as induced expression of the gene by allolactose can be attained. The microorganism having the objective gene may have been modified, for example, so that the activity of BGL is decreased. The microorganism having the objective gene may have been modified, specifically, for example, so that the activity of BGL is decreased as compared with a non-modified strain. The phrase "the activity of BGL is decreased" may mean that at least the allolactose-hydrolyzing activity is decreased. The phrase "the activity of BGL is decreased" may typically mean that both the allolactose-generating activity and the allolactose-hydrolyzing activity are decreased. The activity of BGL can be decreased by, for example, decreasing expression of the BGL gene or disrupting the BGL gene. The activity of BGL can be decreased by, in particular, for example, deleting the BGL gene.

By using induced expression of the objective gene by allolactose, for example, the objective substance can be produced. Specifically, for example, when the objective gene is a gene effective for production of the objective substance, the objective substance can be produced by using induced expression of the objective gene by allolactose. The objective substance may be produced by, for example, inducibly expressing the objective gene during cultivation. The objective substance may also be produced by, for example, using cells in which the objective gene was inducibly expressed. That is, the present invention provides, for example, a method for producing an objective substance, the method comprising a step of culturing a microorganism having an objective gene in a culture medium containing allolactose to thereby generate the objective substance, wherein the objective gene is expressed under control of an allolactose-inducible promoter. Also, the present invention provides, for example, a method for producing an objective substance, the method comprising a step of culturing a microorganism having an objective gene in a culture medium containing allolactose to thereby generate cells of the microorganism, and a step of generating the objective substance by using the cells, wherein the objective gene is expressed under control of an allolactose-inducible promoter. The objective substance may be generated from, for example, a carbon source or a precursor of the objective substance. Previous reports (e.g. WO2018/079687, WO2018/079686, WO2018/079685, WO2018/079684, WO2018/079683, WO2017/073701, WO2018/079705, WO2017/122747, WO2015/060391, WO2018/030507, WO2015/060391, WO2016/104814, WO2015/133547, WO2017/039001, WO2017/033463, WO2017/033464, WO2018/074578, and WO2018/074579) can be used as a reference for production of the objective substance by cultivation or by using cells.

### Examples

Hereinafter, the present invention will be more specifically explained with reference to non-limiting examples.

In the Examples, the term "%" used as a unit for concentration represents "%(w/v)", unless otherwise stated.

### (l) Construction of beta-galactosidase (LacZ)-expressing strain and preparation of beta-galactosidase (LacZ) purified enzyme

PCR was performed by using the genomic DNA of Escherichia coli K-12 MG1655 (ATCC 47076) as the template and primers of SEQ ID NOS: 1 and 2, to amplify a *lacZ* gene fragment encoding beta-galactosidase (LacZ). Separately, PCR was performed by using a plasmid pET24a (Novagen) as the template and primers of SEQ ID NOS: 3 and 4, to amplify an outside fragment of the plasmid. The PCR products were each purified by using Wizard SV Gel and PCR Clean-Up System (Promega). In-Fusion HD Cloning Kit (Takara Bio) was used to ligate the purified PCR products. *E. coli* JM109 was transformed with the reaction product, and cultured on LB agar medium containing 50 mg/L kanamycin at 37°C overnight, to form colonies, to thereby obtain a transformant. A plasmid pET24a-lacZ-His6, into which the fragment of *lacZ* gene encoding beta-galactosidase (LacZ) has been incorporated, was obtained from the obtained transformant by using Wizard Plus Miniprep System (Promega). When using this plasmid, LacZ is expressed in a form added with a His-tag at the C-terminus.

Next, *E. coli* Rosetta(TM) 2(DE3)pLysS Competent Cells (Novagen) were transformed with the plasmid pET24a-lacZ-His6, and cultured on LB agar medium containing 50 mg/L kanamycin at 37°C overnight, to form colonies. The formed colonies were streaked on LB agar medium containing 50 mg/L kanamycin, and single colonies were obtained, to thereby obtain a strain EcLZH6.

The strain EcLZH6 was inoculated to 3 mL of LB medium containing 50 mg/L kanamycin contained in a test tube, and cultured with shaking (120 rpm) at 37°C overnight. The obtained culture broth was inoculated to 50 mL of LB medium containing 50 mg/L kanamycin contained in a shaking flask in a volume of 1/100, and cultured with shaking (120 rpm) at 37°C until OD600 reached 0.8. Then, the shaking flask was moved into a box shaker (13°C), and culture was carried out with shaking (120 rpm) at 13°C for 1 hour. Then, IPTG was added at a concentration of 1 mM, and culture was further continued for 17 hours. After the culture, cells were collected from by centrifugation (4°C, 5000 rpm, 5 min), and frozen for 1 hour in a freezer (-20°C). Then, a soluble protein solution was extracted from the cells by using Ni-NTAFast Start Kit (QIAGEN) under non-denaturing conditions, and LacZ was purified by using a Ni-NTA affinity column, a wash buffer, and an elution buffer included in the kit. A fraction containing LacZ was desalinated and concentrated by using Amicon Ultra-15 30 kDa cut off (Merck Millipore) ultrafiltration membrane filter and 50 mM sodium phosphate buffer (pH 6.5), to obtain a purified enzyme solution of LacZ.

### (2) Allolactose generation from lactose by beta-galactosidase (LacZ) purified enzyme

Allolactose generation from lactose in vitro was confirmed using the LacZ purified enzyme solution obtained in (1) above.

That is, 50 mM sodium phosphate buffer (pH=6.5) containing 2% or 20% lactose was used as a substrate solution, and the LacZ purified enzyme solution was added thereto at a concentration of 30 ng/ml in terms of protein concentration, and the reaction was carried out at 37°C. The reaction mixture was sampled over time and subjected to heating at 95°C for 10 min to inactivate the enzyme, and the supernatant was passed through a 0.22 µm pore size filter, to obtain samples. The samples were subjected to ion exchange chromatography to analyze and quantify the products. The conditions for ion-exchange chromatography are shown in Tables 1 and 2.

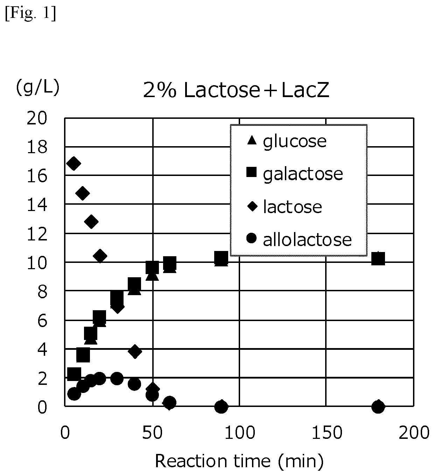

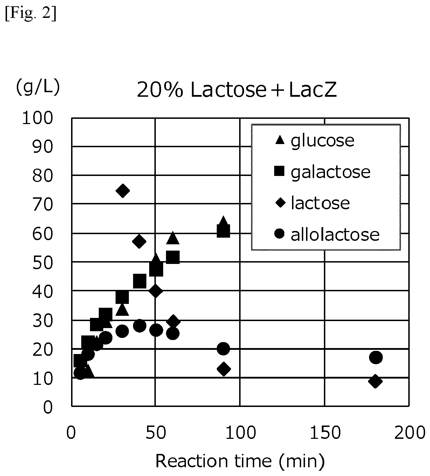

The results are shown in Figs. 1 and 2. Allolactose generation was observed both in the 2% lactose solution and the 20% lactose solution. In the 2% lactose solution, a maximum of about 2 g/L of allolactose was generated, followed by a decrease in allolactose concentration (Fig. 1). In the 20% lactose solution, a maximum of about 28 g/L of allolactose was generated, followed by a decrease in allolactose concentration (Fig. 2). These results indicated that allolactose is generated from lactose by LacZ, that the generation concentration and generation yield of allolactose are increased with increasing the concentration of the substrate lactose, and that generated allolactose is degraded. The generation yield of allolactose from lactose by LacZ was approximately 14% even under the condition of using the 20% lactose solution having a higher substrate concentration.

**Table 1**

| | |
|---|---|
| Analysis column | Dionex ISC-3000 |
| Column | Carbo Pac PA1 Analytical (2.0 mm ×250 mm) |
| Guard column | Carbo Pac PA1 Guard (2.0 mm ×50 mm) |
| Mobile phase | A H₂O |
| | B 0.1 M NaOH |
| | C 0.5 M CH₃COONa/0.1 M NaOH |
| Gradient pattern | As described below |
| Column temperature | 30°C |
| Flow rate | 0.25 ml/min |
| Detector | Electrochemical detection (PAD) |
| Injection volume | 5.0 µl |

**Table 2**

| Gradient pattern of mobile phase | | | |
|---|---|---|---|
| Time (min) | A (%) | B (%) | C (%) |
| -30 | 0 | 100 | 0 |
| -20 | 0 | 100 | 0 |
| 0 | 90 | 10 | 0 |
| 25 | 90 | 10 | 0 |
| 40 | 36 | 64 | 0 |
| 50 | 0 | 88 | 12 |
| 70 | 0 | 64 | 36 |
| 71 | 0 | 0 | 100 |
| 80 | 0 | 0 | 100 |

### (3) Evaluation of effect of glucose on allolactose generation from lactose by beta-galactosidase (LacZ) purified enzyme

The effect of glucose on allolactose generation from lactose in vitro was evaluated using the LacZ purified enzyme solution obtained in (1) above.

That is, 50 mM sodium phosphate buffer (pH=6.5) containing 10% or 20% glucose in addition to 20% lactose was used as a substrate solution, and the LacZ purified enzyme solution was added thereto at a concentration of 30 ng/ml in terms of protein concentration, and the reaction was carried out at 37°C. The reaction mixture was sampled over time, and the products were analyzed using the same procedure as (2) above.

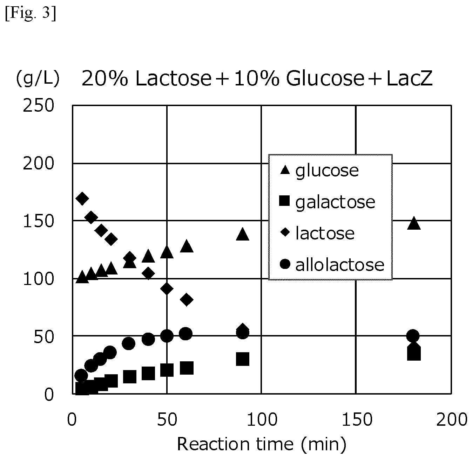

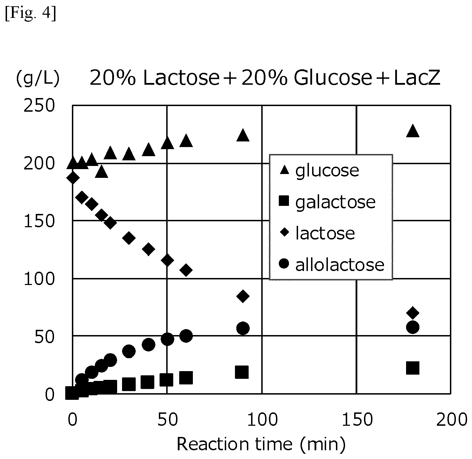

The results are shown in Figs. 3 and 4. As a result, in both the cases of using the substrate solutions containing 10% and 20% glucose in addition to 20% lactose, the generation concentration of allolactose exceeded 50 g/L, and the generation yield of allolactose from lactose was increased significantly from approximately 14% observed in the case of not adding glucose to over 25%. In addition, in both the cases of using the substrate solutions containing 10% and 20% glucose in addition to 20% lactose, inhibition of allolactose degradation was observed. That is, it is considered that hydrolysis of allolactose by beta-galactosidase (LacZ) was inhibited by product inhibition by glucose. In addition, strong inhibition of allolactose degradation was observed in the case of using the substrate solution containing 20% glucose as compared with the case of using the substrate solution containing 10% glucose. That is, it was revealed that a higher concentration of glucose is desirable to inhibit allolactose degradation.

### (4) Allolactose generation from lactose by E. coli viable cells having beta-galactosidase (LacZ)

Use of beta-galactosidase (LacZ) purified enzyme requires time and cost for purification of the enzyme or the like. Therefore, the inventors attempted to efficiently generate allolactose from lactose in vitro using untreated *E. coli* viable cells having beta-galactosidase (LacZ).

*E. coli* viable cells prepared by cultivating the EcLZH6 strain by the method described in (1) above were added to the 10 substrate solutions listed in Table 3 to achieve OD₆₀₀=5.0, and stored at room temperature (26°C), to allow the reaction to proceed. The reaction mixture was sampled over time, and the products were analyzed using the same procedure as (2) above.

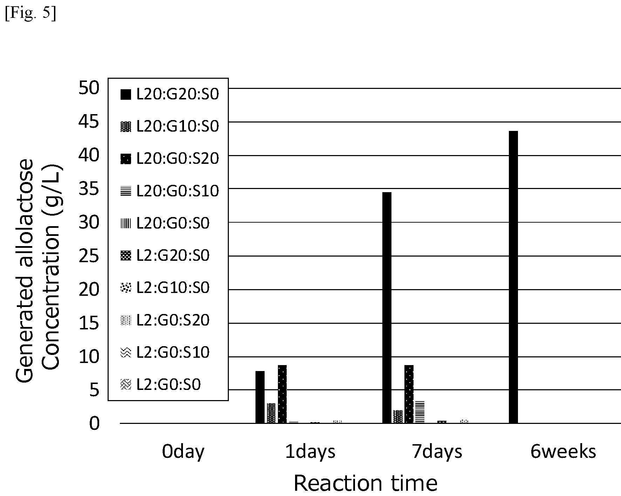

The results are shown in Fig. 5. Accumulation of allolactose was observed mainly in the cases of using the substrate solutions containing 20% lactose. In particular, a significant accumulation of allolactose was observed in the substrate solution containing 20% glucose in addition to 20% lactose. Although allolactose generation was observed also in the case of using the substrate solution containing 20% sorbitol in addition to 20% lactose, the generation concentration of allolactose was relatively low, and allolactose disappeared after a long period (6 weeks) of storage. By contrast, accumulation of allolactose was hardly detected in the case of using the substrate solution containing only 20% lactose. These results revealed that including glucose in the substrate solution was effective than sorbitol for production of allolactose from lactose using untreated viable cells having beta-galactosidase (LacZ), and that increasing the concentrations of lactose and glucose in the substrate solution was effective. From the above, it is considered that inhibition of the hydrolysis reaction of beta-galactosidase (LacZ) by product inhibition by glucose (e.g. inhibition of degradation of allolactose and/or lactose) and elution of intracellular components including enzymes from viable cells under high osmotic conditions possibly synergistically improved generation and accumulation of allolactose. Hence, it can be effective to mimic high osmotic conditions by addition of other component(s) or to use other component(s) that cause product inhibition of the hydrolysis reaction of beta-galactosidase (LacZ) such as galactose.

**Table 3**

| Notation in figures | Lactose | Glucose | Sorbitol |
|---|---|---|---|
| L20:G20:S0 | 20% | 20% | 0 |
| L20:G10:S0 | 20% | 10% | 0 |
| L20:G0:S20 | 20% | 0 | 20% |
| L20:G0:S10 | 20% | 0 | 10% |
| L20:G0:S0 | 20% | 0 | 0 |
| L2:G20:S0 | 2% | 20% | 0 |
| L2:G10:S0 | 2% | 10% | 0 |
| L2:G0:S20 | 2% | 0 | 20% |
| L0:G0:S10 | 2% | 0 | 10% |
| L0:G0:S0 | 2% | 0 | 0 |

### (5) Evaluation of the effect of temperature on allolactose generation from lactose by E. coli viable cells having beta-galactosidase (LacZ)

The effect of temperature on allolactose generation from lactose in vitro was evaluated using untreated *E. coli* viable cells having beta-galactosidase (LacZ).

*E. coli* viable cells prepared by cultivating the EcLZH6 strain by the method described in (1) above were added to the substrate solution of L20:G20:S0 of Table 3 to achieve OD₆₀₀=10, and stored at room temperature (26°C), 37°C, or 42°C to allow the reaction to proceed. The reaction mixture was sampled over time, and the products were analyzed using the same procedure as (2) above.

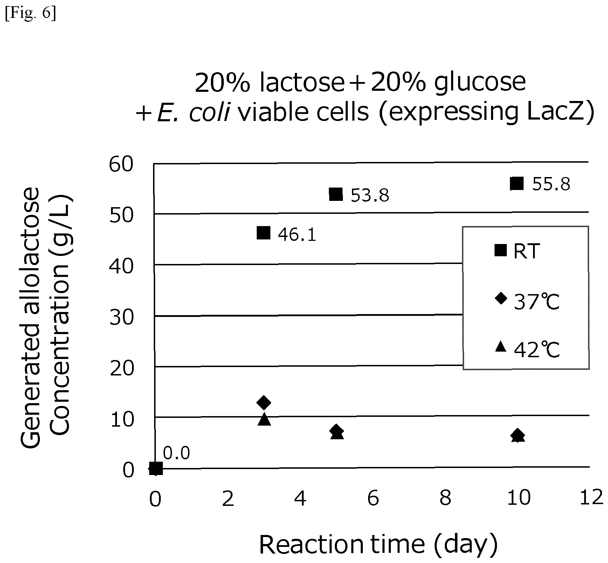

The results are shown in Fig. 6. Accumulation concentration of allolactose decreased relatively quickly when the reaction was allowed to proceed at 37°C or 42°C. By contrast, decrease in accumulation concentration of allolactose was not observed when the reaction was allowed to proceed at room temperature (26°C). These results revealed that it is more effective to proceed with the reaction at a low temperature such as room temperature (26°C) than at a high temperature for maintaining accumulation of allolactose at a high concentration of allolactose for a long time.

### (6) Evaluation of lac promoter inducibility of prepared allolactose 1

The lac promoter inducibility of allolactose was evaluated by the Blue-White screening method using *E. coli* JM109 and pUC19 plasmid.

Competent cells of *E. coli* JM109 (Takara Bio) were transformed with pUC19 plasmid (Takara Bio), and cultured on LB agar medium containing 50 mg/L ampicillin at 37°C overnight, to form colonies. The formed colonies were streaked on LB agar medium containing 50 mg/L ampicillin, and single colonies were obtained, to thereby obtain a strain JM109+pUC19. This strain exhibits beta-galactosidase activity due to alpha-complementarity by a part of LacZ enzymes (LacZα) expressed from the lac promoter on pUC19 plasmid. That is, this strain exhibits beta-galactosidase activity when gene expression from the lac promoter is induced. Beta-galactosidase activity of the strain JM109 is lost unless alpha-complementarity is exerted.

The reaction mixture containing 55.8 g/L of allolactose obtained in (4) above was subjected to heating at 95°C for 10 minutes to inactivate the enzyme, and the supernatant was passed through a 0.22 µm pore size filter, to obtain an allolactose solution. The obtained allolactose solution was diluted according to the dilution factors listed in Table 4 and added to LB agar media, and X-Gal (Thermo Fisher Scientific) was further added, to prepare LB agar media for Blue-White screening containing various concentrations of allolactose and X-gal. X-gal is degraded by beta-galactosidase, and the degradation product thereof, 4-chloro-3-bromo-indigo, provides blue coloration.

The JM109+pUC19 strain was streaked on each LB agar medium for Blue-White screening and cultured at 37°C overnight, and the coloration of single-isolated colonies was observed.

The results are shown in Table 4. In the table, "× (cross)" indicates no blue coloration, "△ (triangle)" indicates unclear blue coloration, and "∘ (circle)" indicates clear blue coloration. In particular, under conditions with allolactose concentrations of 0.03 to 1.5 mM, blue coloration of colonies was observed, i.e., induction of gene expression from the lac promoter on the pUC19 plasmid. By contrast, under conditions with allolactose concentrations of 0.0075 to 0.015 mM, blue coloration was obscured, i.e., it was suggested that the lac promoter inducibility of allolactose was attenuated.

**Table 4**

| Final concentration of allolactose in plate media (mM) | Dilution factor of prepared allolactose-containing solution (163 mM, 55.8 g/L) | Blue coloration of formed colonies of JM109+pUC19 strain |
|---|---|---|
| 15 | 11 | × |
| 3 | 54 | △ |
| 1.5 | 109 | ○ |
| 0.3 | 543 | ○ |
| 0.15 | 1087 | ○ |
| 0.075 | 2174 | ○ |
| 0.03 | 5434 | ○ |
| 0.015 | 10868 | △ |
| 0.0075 | 21736 | △ |

### (6) Evaluation of lac promoter inducibility of prepared allolactose 2

It is assumed that since blue coloration of the degradation product of X-gal by beta-galactosidase (LacZ) was used as the evaluation index in the evaluation in (5) above, allolactose was degraded by beta-galactosidase (LacZ), which is the reporter gene, and as a result the lac promoter inducibility of allolactose was attenuated under conditions with allolactose concentrations of 0.0075 to 0.015 mM Therefore, the reporter gene was changed to DasherGFP, which is a fluorescent protein, instead of beta-galactosidase, and the lac promoter inducibility of allolactose in strains that do not degrade allolactose was evaluated.

PCR was performed by using FPB-27-441 plasmid (Cosmo Bio) as the template and primers of SEQ ID NOS: 5 and 6, to amplify a gene fragment encoding DasherGFP. Separately, PCR was performed by using a plasmid pUC19 plasmid (Takara Bio) as the template and primers of SEQ ID NOS: 7 and 8, to amplify an outside fragment of the plasmid. The PCR products were each purified by using Wizard SV Gel and PCR Clean-Up System (Promega). In-Fusion HD Cloning Kit (Takara Bio) was used to ligate the purified PCR products. *E. coli* JM109 was transformed with the reaction product, and cultured on LB agar medium containing 50 mg/L ampicillin at 37°C overnight, to form colonies, to thereby obtain a transformant. A plasmid pUC 19-DGFP, in which *LacZα* gene has been replaced with *DasherGFP* gene, was obtained from the obtained transformant by using Wizard Plus Miniprep System (Promega). Next, PCR was performed by using the genomic DNA of Escherichia coli K-12 MG1655 (ATCC 47076) as the template and primers of SEQ ID NOS: 9 and 10, to amplify a *lacI* gene fragment encoding a LacI repressor. Separately, PCR was performed by using the plasmid pUC19-DGFP as the template and primers of SEQ ID NOS: 11 and 12, to amplify an outside fragment of the plasmid. The PCR products were each purified by using Wizard SV Gel and PCR Clean-Up System (Promega). In-Fusion HD Cloning Kit (Takara Bio) was used to ligate the purified PCR products. *E. coli* JM109 was transformed with the reaction product, and cultured on LB agar medium containing 50 mg/L ampicillin at 37°C overnight, to form colonies, to thereby obtain a transformant. A plasmid pUC19-DGFP-PtI, in which *LacZα* gene has been replaced with *DasherGFP* gene and into which Ptac-LacI has been incorporated, was obtained from the obtained transformant by using Wizard Plus Miniprep System (Promega). Ptac-LacI was incorporated for the purpose of strongly suppressing expression of DasherGFP upon not inducing the lac promoter, and thereby facilitating observation of fluorescent coloration of DasherGFP upon not inducing the lac promoter.

Competent cells of *E. coli* JM109 (Takara Bio) were transformed with the plasmid pUC19-DGFP-PtI, and cultured on LB agar medium containing 50 mg/L ampicillin at 37°C overnight, to form colonies. The formed colonies were streaked on LB agar medium containing 50 mg/L ampicillin, and single colonies were obtained, to thereby obtain a strain JM109+pUC-DGFP-PtI.

The allolactose solution obtained in (5) above was 21736-fold diluted and added to LB agar medium, to prepare LB agar medium containing 0.0075 mM allolactose.

The JM109+pUC-DGFP-PtI strain was streaked on LB agar medium containing 0.0075 mM allolactose and LB agar medium not containing allolactose and cultured at 37°C overnight, and single-isolated colonies were obtained. As a comparison, the JM109+pUC19 strains strain was streaked on LB agar medium and cultured at 37°C overnight, and colonies were single-isolated. Fluorescent coloration of the colonies obtained under each condition was observed with a UV illuminator.

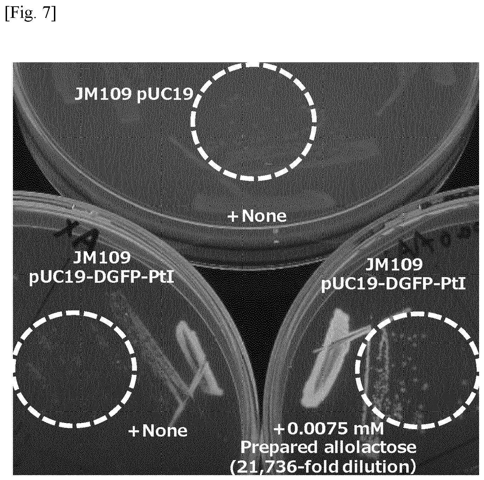

The results are shown in Fig. 7. Even under the condition containing allolactose at 0.0075 mM, fluorescent coloration of the colonies of the JM109+pUC-DGFP-PtI strain was observed. That is, it was revealed that allolactose is able to induce the lac promoter on the pUC19 plasmid even at a very low concentration under conditions where allolactose degradation by beta-galactosidase does not occur. These results suggest that, when allolactose is used to induce the lac promoter, it is effective to delete the activity of beta-galactosidase activity, which is a degrading enzyme.

### (7) Evaluation of lac promoter inducibility of prepared allolactose 3

The lac promoter inducibility of allolactose was evaluated using the inducibility of pET expression system, which is commonly used for heterologous protein production, as an indicator. In the pET expression system, T7-polymerase, which is inducibly expressed from the lac promoter, highly expresses a gene downstream of the T7 promoter (e.g. a gene encoding a heterologous protein). As the heterologous protein to be produced, beta-galactosidase gene of a filamentous fungus Talaromyces cellulolyticus (hereinafter, also referred to as "*gh1-2* gene") was used.

RNA was prepared form cells of the *T. cellulolyticus* strain Y-94 (CBS 136886) by using RNeasy Plant Mini Kit (QIAGEN), and a full-length cDNA solution was prepared form the RNA solution by using SMARTer (R) RACE 5'/3' kit. PCR was performed by using the cDNA solution as the template and primers of SEQ ID NOS: 13 and 14, to amplify a cDNA fragment of *gh1-2* gene. Separately, PCR was performed by using a plasmid pET24a (Novagen) as the template and primers of SEQ ID NOS: 3 and 4, to amplify an outside fragment of the plasmid. The PCR products were each purified by using Wizard SV Gel and PCR Clean-Up System (Promega). In-Fusion HD Cloning Kit (Takara Bio) was used to ligate the purified PCR products. *E. coli* JM109 was transformed with the reaction product, and cultured on LB agar medium containing 50 mg/L kanamycin at 37°C overnight, to form colonies, to thereby obtain a transformant. A plasmid pET24a-gh1-2-His6, into which the cDNA fragment of *gh1-2* gene has been incorporated, was obtained from the obtained transformant by using Wizard Plus Miniprep System (Promega).

*E. coli* Rosetta(TM) 2(DE3)pLysS Competent Cells (Novagen) were transformed with the plasmid ET24a-gh1-2-His6, and cultured on LB agar medium containing 50 mg/L kanamycin at 37°C overnight, to form colonies. The formed colonies were streaked on LB agar medium containing 50 mg/L kanamycin, and single colonies were obtained, to thereby obtain a strain EcGHH6.

The strain EcGHH6 was inoculated to 3 mL of LB medium containing 50 mg/L kanamycin contained in a test tube, and cultured with shaking (120 rpm) at 37°C overnight. The obtained culture broth was inoculated to 50 mL of LB medium containing 50 mg/L kanamycin contained in a shaking flask in a volume of 1/100, and cultured with shaking (120 rpm) at 37°C until OD600 reached 0.8. Then, the shaking flask was moved into a box shaker (13°C), and culture was carried out with shaking (120 rpm) at 13°C for 1 hour. Then, IPTG was added at a final concentration of 1 mM or the allolactose solution obtained in (5) above was added at a final concentration of 1 mM, 0.1 mM, 0.01 mM, 0,001 mM, or 0.0001 mM, and culture was further continued for 14 hours. After the culture, cells were collected from by centrifugation (4°C, 5000 rpm, 5 min), and 5 µl of a 10-fold concentrated cell suspension was subject to SDS-PAGE.

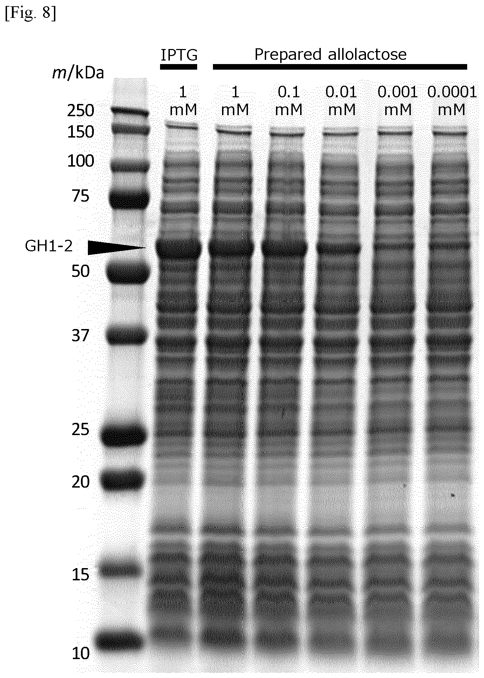

The results are shown in Fig. 8. It was revealed that expression of a heterologous protein is attained also in a liquid culture by induction with allolactose at a final concentration of 0.1 mM almost equally to the case of inducing expression of T7-polymerase under the lac promoter by 1 mM IPTG. It was suggested that expression level of a heterologous protein decreased due to decrease in allolactose concentration at allolactose concentrations below 0.1 mM The *LacZ* gene encoding beta-galactosidase has not been disrupted in the EcGHH6 strain. Based on the results of (6) above, it is considered that expression of a heterologous protein is sufficiently induced even at allolactose concentrations below 0.1 mM when LacZ is disrupted in the heterologous protein-producing strain.

### Industrial Applicability

According to the present invention, allolactose can be efficiently produced.

### <Explanation of Sequence Listing>

SEQ ID NOS:
1-14: Primers
15: Nucleotide sequence of *lacZ* gene of *E*. *coli* K-12 MG1655
16: Amino acid sequence of LacZ protein of *E. coli* K-12 MG1655

## Claims

1. A method for producing allolactose, the method comprising a step (A) mentioned below:
(A) a step of bringing cells of a microorganism having beta-galactosidase into contact with lactose in the presence of a substance X that inhibits hydrolysis of allolactose,
wherein the cells have not been subject to a treatment for increasing membrane permeability, and
wherein the total concentration of lactose and the substance X is 1 Eq/L or more.

2. A method for producing a gene expression inducer, the method comprising a step (A) mentioned below:
(A) a step of bringing cells of a microorganism having beta-galactosidase into contact with lactose in the presence of a substance X that inhibits hydrolysis of allolactose,
wherein the cells have not been subject to a treatment for increasing membrane permeability, and
wherein the total concentration of lactose and the substance X is 1 Eq/L or more.

3. The method according to claim 1 or 2, wherein the substance X is glucose and/or galactose.

4. The method according to any one of claims 1 to 3, wherein the substance X is glucose.

5. The method according to any one of claims 1 to 4, wherein the concentration of lactose in the step (A) is 120 g/L or more.

6. The method according to any one of claims 1 to 5, wherein the concentration of the substance X in the step (A) is 120 g/L or more.

7. The method according to any one of claims 1 to 6, wherein the step (A) is carried out at a temperature of 30°C or below.

8. The method according to any one of claims 1 to 7, wherein the cells have not been subject to any of drying treatment, freeze-thaw treatment, surfactant treatment, and organic solvent treatment.

9. The method according to any one of claims 1 to 8, wherein the microorganism is *Escherichia coli.*

10. The method according to any one of claims 1 to 9, wherein the microorganism has been modified so that the activity of beta-galactosidase is increased as compared with a non-modified strain.

11. The method according to any one of claims 1 to 10, wherein the beta-galactosidase is a protein defined in (a), (b), or (c) mentioned below:
(a) a protein comprising the amino acid sequence of SEQ ID NO: 16;
(b) a protein comprising the amino acid sequence of SEQ ID NO: 16, but which includes substitution, deletion, insertion, and/or addition of 1 to 10 amino acid residues, and having allolactose-generating activity and allolactose-hydrolyzing activity;
(c) a protein comprising an amino acid sequence showing an identity of 90% or higher to the amino acid sequence of SEQ ID NO: 16, and having allolactose-generating activity and allolactose-hydrolyzing activity.
